(19) Europäisches Patentamt
European Patent Office
Office européen des brevets

(11) EP 4 230 653 A1

(12) **EUROPEAN PATENT APPLICATION**
published in accordance with Art. 153(4) EPC

(43) Date of publication:
**23.08.2023 Bulletin 2023/34**

(21) Application number: **21879468.3**

(22) Date of filing: **14.10.2021**

(51) International Patent Classification (IPC):
***C07K 16/28*** *(2006.01)* ***A61K 47/65*** *(2017.01)*
***A61K 47/68*** *(2017.01)* ***A61K 39/395*** *(2006.01)*
***A61P 35/00*** *(2006.01)*

(52) Cooperative Patent Classification (CPC):
**A61K 39/395; A61K 47/65; A61K 47/68; A61P 35/00; C07K 16/28**

(86) International application number:
**PCT/CN2021/123733**

(87) International publication number:
**WO 2022/078425 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.10.2020 CN 202011097383**
**08.10.2021 CN 202111171200**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **YANG, Yang**
  **Shanghai 200245 (CN)**
- **YU, Jia**
  **Shanghai 200245 (CN)**
- **TAO, Weikang**
  **Shanghai 200245 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) ANTI-HER3 ANTIBODY AND ANTI-HER3 ANTIBODY-DRUG CONJUGATE AND MEDICAL USE THEREOF

(57) Provided are an anti-HER3 antibody and an anti-HER3 antibody-drug conjugate and a medical use thereof, specifically, the anti-HER3 antibody, and the anti-HER3 antibody-drug conjugate as represented by general formula (Pc-L-Y-D), wherein Pc is an anti-HER3 antibody, and L, Y and n are as defined in the description.

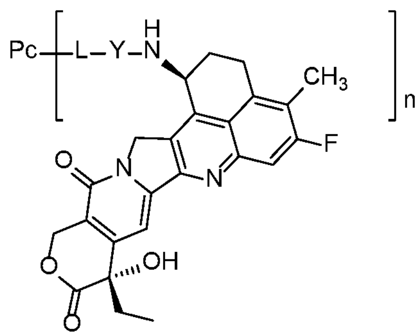

(Pc-L-Y-D)

EP 4 230 653 A1

## Description

### TECHNICAL FIELD

The present disclosure relates to an anti-HER3 antibody and an anti-HER3 antibody-exatecan analog conjugate, a preparation method for the same, a pharmaceutical composition comprising the same, and use of the same in preparing a medicament for treating a HER3-mediated disease or disorder, particularly in preparing an anti-cancer medicament.

### BACKGROUND

The statements herein merely provide background information related to the present disclosure and may not necessarily constitute the prior art.

HER3 (epidermal growth factor receptor3, ErbB-3 or HER3) is a member of the epidermal growth factor receptor (EGFR) family. This family includes HER1 (erbB 1, EGFR), HER2 (erbB2, NEU), HER3 (erbB3) and HER4 (erbB4). These receptors each comprise 3 parts: an extracellular region, a transmembrane region and an intracellular region. The extracellular region comprises 4 domains. The intracellular region comprises one intracellular tyrosine kinase domain for signaling and one tail in the cytoplasm comprising tyrosine phosphorylation residues. When a ligand binds to extracellular domains I and III, cell signaling is initiated. Normally, these receptors mediate cell division, migration, survival and organ development. When the EGFR family members mutate, the aberrant signaling caused by them stimulates cell survival, associated with cancer progression. The basic principle behind the activation and physiological action of the HER3 receptor is similar to those of the other family members, except that its ligands include neuregulin 1 (NRG-1) and neuregulin 2 (NRG-2) and that activated HER3 is unable to form a homomer but only a heterodimer with EGFR or HER2. In the process of HER3 forming a heterodimer, its intracellular domain exhibits higher tyrosine phosphorylase activity. Structural analysis shows that the intracellular domain of HER3 has six P85 (PI-3K subunit) binding sites. This specific structure determines that HER3, when interacting with P85 regulatory subunits, can recruit up to six PI-3K to the regulatory subunit sites, thereby strongly activating the PI-3K signaling pathway. In fact, the HER3/HER2 dimer is the most active of the HER dimers. EGFRs are widely distributed on the surface of the cells such as mammalian epithelial cells, fibroblasts, glial cells and keratinocytes. The EGFR signaling pathway plays an important role in physiological processes such as cell growth, proliferation and differentiation.

HER3 is highly expressed in various common malignancies such as breast cancer, gastric cancer, ovarian cancer, prostate cancer, bladder cancer, colorectal cancer, head and neck squamous cell carcinoma and melanoma. The HER3 gene rarely mutates. Different from the high-level expression or overactivation caused by mutations in the EGFR gene, the high-level expression of HER3 is mainly caused by increases in mRNA transcription which lead to increases in protein translation, and is generally accompanied by the high-level expression of HER2. The high-level expression of HER3 is closely related to the development and progression of many tumors, as well as the survival of subjects. Therefore, the research on anti-tumor drugs targeting HER3 is of great significance.

### SUMMARY

The present disclosure relates to an anti-HER3 antibody, an anti-HER3 antibody-exatecan analog conjugate and use thereof.

The present disclosure provides an isolated anti-HER3 antibody, wherein the anti-HER3 antibody has one or more of the following characteristics:

a. the anti-HER3 antibody binds to HER3 protein with an apparent affinity $EC_{50}$ of less than 0.5 nM, as determined by ELISA;
b. the anti-HER3 antibody binds to HER3 protein expressed by MCF7 cells with an apparent affinity $EC_{50}$ of less than 0.2 nM, as determined by FACS;
c. the anti-HER3 antibody can be endocytosed by cells expressing human HER3.

The present disclosure provides an isolated anti-HER3 antibody, wherein the anti-HER3 antibody has one or more of the following characteristics:

a. the anti-HER3 antibody binds to HER3 protein with an apparent affinity $EC_{50}$ of less than 0.5 nM, as determined by ELISA;
b. the anti-HER3 antibody binds to HER3 protein expressed by MCF7 cells with an apparent affinity $EC_{50}$ of less than 0.2 nM, as determined by FACS;
c. the anti-HER3 antibody can be endocytosed by cells expressing human HER3; the anti-HER3 antibody has an

$IC_{50}$ of less than 2 nM, as determined using the method of Test Example 3;
d. the anti-HER3 antibody can be endocytosed by cells expressing human HER3; preferably, the anti-HER3 antibody has an FITC signal of greater than 300, as determined using the method of Test Example 4.

In some embodiments, the anti-HER3 antibody according to any one of the above comprises: (1) HCDR1, HCDR2 and HCDR3 comprised in a heavy chain variable region set forth in SEQ ID NO: 7; and (2) LCDR1, LCDR2 and LCDR3 comprised in a light chain variable region set forth in SEQ ID NO: 8.
In some embodiments, the anti-HER3 antibody according to any one of the above comprises a heavy chain variable region and a light chain variable region, wherein:

a. the heavy chain variable region comprises HCDR1, HCDR2 and HCDR3 set forth in SEQ ID NO: 9, SEQ ID NO: 10 and SEQ ID NO: 11, respectively, and the light chain variable region comprises LCDR1, LCDR2 and LCDR3 set forth in SEQ ID NO: 12, SEQ ID NO: 13 and SEQ ID NO: 14, respectively;

wherein the CDR regions are defined according to the Chothia numbering scheme.
In some embodiments, the anti-HER3 antibody according to any one of the above comprises a heavy chain variable region and a light chain variable region, wherein:

b. the heavy chain variable region comprises HCDR1, HCDR2 and HCDR3 set forth in SEQ ID NO: 15, SEQ ID NO: 16 and SEQ ID NO: 17, respectively, and the light chain variable region comprises LCDR1, LCDR2 and LCDR3 set forth in SEQ ID NO: 18, SEQ ID NO: 19 and SEQ ID NO: 20, respectively;
wherein the CDR regions are defined according to the IMGT numbering scheme.

In some embodiments, the anti-HER3 antibody according to any one of the above comprises a heavy chain variable region and a light chain variable region, wherein:

c. the heavy chain variable region comprises HCDR1, HCDR2 and HCDR3 set forth in SEQ ID NO: 21, SEQ ID NO: 22 and SEQ ID NO: 23, respectively, and the light chain variable region comprises LCDR1, LCDR2 and LCDR3 set forth in SEQ ID NO: 24, SEQ ID NO: 25 and SEQ ID NO: 26, respectively;
wherein the CDR regions are defined according to the Kabat numbering scheme. The present disclosure provides an isolated anti-HER3 antibody, wherein the anti-HER3 antibody comprises a heavy chain variable region and a light chain variable region,

wherein:

a. the heavy chain variable region comprises HCDR1, HCDR2 and HCDR3 set forth in SEQ ID NO: 9, SEQ ID NO: 10 and SEQ ID NO: 11, respectively, and the light chain variable region comprises LCDR1, LCDR2 and LCDR3 set forth in SEQ ID NO: 12, SEQ ID NO: 13 and SEQ ID NO: 14, respectively;

wherein the CDR regions are defined according to the Chothia numbering scheme.
The present disclosure provides an isolated anti-HER3 antibody, wherein the anti-HER3 antibody comprises a heavy chain variable region and a light chain variable region, wherein:

b. the heavy chain variable region comprises HCDR1, HCDR2 and HCDR3 set forth in SEQ ID NO: 15, SEQ ID NO: 16 and SEQ ID NO: 17, respectively, and the light chain variable region comprises LCDR1, LCDR2 and LCDR3 set forth in SEQ ID NO: 18, SEQ ID NO: 19 and SEQ ID NO: 20, respectively;
wherein the CDR regions are defined according to the IMGT numbering scheme.

The present disclosure provides an isolated anti-HER3 antibody, wherein the anti-HER3 antibody comprises a heavy chain variable region and a light chain variable region, wherein:

c. the heavy chain variable region comprises HCDR1, HCDR2 and HCDR3 set forth in SEQ ID NO: 21, SEQ ID NO: 22 and SEQ ID NO: 23, respectively, and the light chain variable region comprises LCDR1, LCDR2 and LCDR3 set forth in SEQ ID NO: 24, SEQ ID NO: 25 and SEQ ID NO: 26, respectively;
wherein the CDR regions are defined according to the Kabat numbering scheme.

In some embodiments, the anti-HER3 antibody according to any one of the above is a human antibody or an antigen-binding fragment.

In some embodiments, the anti-HER3 antibody according to any one of the above comprises a heavy chain variable region and a light chain variable region, wherein:
the heavy chain variable region has an amino acid sequence having at least 90%, 91%, 92%, 93%, 94%, 95%, 96%, 97%, 98%, 99%, or 100% sequence identity to SEQ ID NO: 7, and/or the light chain variable region has an amino acid sequence having at least 90%, 91%, 92%, 93%, 94%, 95%, 96%, 97%, 98%, 99% or 100% sequence identity to SEQ ID NO: 8.
In some embodiments, the anti-HER3 antibody according to any one of the above comprises a heavy chain variable region and a light chain variable region, wherein:

the heavy chain variable region has an amino acid sequence set forth in SEQ ID NO: 7, and the light chain variable region has an amino acid sequence set forth in SEQ ID NO: 8; or
in some embodiments, the anti-HER3 antibody according to any one of the above further comprises an antibody heavy chain constant region and an antibody light chain constant region; preferably, the heavy chain constant region is selected from the group consisting of constant regions of human IgG1, IgG2, IgG3 and IgG4 and conventional variants thereof, and the light chain constant region is selected from the group consisting of constant regions of human antibody κ and λ chains and conventional variants thereof; more preferably, the antibody comprises a heavy chain constant region set forth in SEQ ID NO: 5 and a light chain constant region set forth in SEQ ID NO: 6.

In some embodiments, the anti-HER3 antibody according to any one of the above comprises:
a heavy chain having at least 85%, 90%, 91%, 92%, 93%, 94%, 95%, 96%, 97%, 98%, 99% or 100% sequence identity to SEQ ID NO: 27, and/or a light chain having at least 85%, 90%, 91%, 92%, 93%, 94%, 95%, 96%, 97%, 98%, 99% or 100% sequence identity to SEQ ID NO: 28.
In some embodiments, the anti-HER3 antibody according to any one of the above comprises:
a heavy chain set forth in SEQ ID NO: 27 and a light chain set forth in SEQ ID NO: 28.
In some embodiments, the anti-HER3 antibody according to any one of the above has one or more of the following characteristics:

a. the anti-HER3 antibody binds to HER3 protein with an apparent affinity EC50 of less than 0.5 nM, as determined by ELISA;
b. the anti-HER3 antibody binds to HER3 protein expressed by MCF7 cells with an apparent affinity EC50 of less than 0.2 nM, as determined by FACS;
c. the anti-HER3 antibody can be endocytosed by cells expressing human HER3.

In some embodiments, the anti-HER3 antibody according to any one of the above has one or more of the following characteristics:

a. the anti-HER3 antibody binds to HER3 protein with an apparent affinity EC50 of less than 0.5 nM, as determined by ELISA;
b. the anti-HER3 antibody binds to HER3 protein expressed by MCF7 cells with an apparent affinity EC50 of less than 0.2 nM, as determined by FACS;
c. the anti-HER3 antibody can be endocytosed by cells expressing human HER3; the anti-HER3 antibody has an IC50 of less than 2 nM, as determined using the method of Test Example 3;
d. the anti-HER3 antibody can be endocytosed by cells expressing human HER3; preferably, the anti-HER3 antibody has an FITC signal of greater than 300, as determined using the method of Test Example 4.

In some embodiments, the present disclosure also provides an isolated anti-HER3 antibody, wherein the antibody competes for binding to human HER3 with the anti-HER3 antibody according to any one of the above.
In some embodiments, the present disclosure also provides a nucleic acid molecule encoding the anti-HER3 antibody according to any one of the above.
In some embodiments, the present disclosure also provides a host cell comprising the nucleic acid molecule according to any one of the above.
In some embodiments, the present disclosure also provides a pharmaceutical composition comprising a therapeutically effective amount of the anti-HER3 antibody according to any one of the above, or the nucleic acid molecule described above, and one or more pharmaceutically acceptable carriers, diluents or excipients.
In some embodiments, the present disclosure also provides an immunoconjugate comprising the anti-HER3 antibody according to any one of the above and an effector molecule, wherein the effector molecule is coupled to the anti-HER3 antibody; preferably, the effector molecule is selected from the group consisting of a radioisotope, an anti-tumor agent, an immunomodulator, a biological response modifier, a lectin, a cytotoxic drug, a chromophore, a fluorophore, a chemiluminescent compound, an enzyme, a metal ion, and any combination thereof.

In some embodiments, the present disclosure also provides a method for immunodetection or determination of HER3 comprising a step of contacting the anti-HER3 antibody according to any one of the above with a subject or a sample from the subj ect.

In some embodiments, the present disclosure also provides an antibody-drug conjugate of general formula (Pc-L-Y-D) or a pharmaceutically acceptable salt thereof:

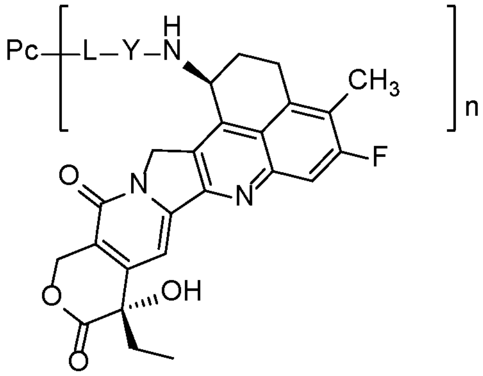

(Pc-L-Y-D)

wherein:

Y is selected from the group consisting of -O-($CR^aR^b$)$_m$-$CR^1R^2$-C(O)-, -O-$CR^1R^2$-($CR^aR^b$)$_m$-, -O-$CR^1R^2$-, -NH-($CR^aR^b$)$_m$-$CR^1R^2$-C(O)- and -S-($CR^aR^b$)$_m$-$CR^1R^2$-C(O)-;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or, $R^a$ and $R^b$, together with the carbon atom to which they are attached, form cycloalkyl or heterocyclyl; $R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with the carbon atom to which they are attached, form cycloalkyl or heterocyclyl;

or, $R^a$ and $R^2$, together with the carbon atom to which they are attached, form cycloalkyl or heterocyclyl;

m is an integer from 0 to 4;

n is a decimal or an integer from 1 to 10;

L is a linker unit;

Pc is the anti-HER3 antibody according to any one of the above.

In some embodiments, in the antibody-drug conjugate of general formula (Pc-L-Y-D) or the pharmaceutically acceptable salt thereof according to any one of the above, n is a decimal or an integer from 1 to 8. In some embodiments, n is a decimal or an integer from 3 to 8.

In some embodiments, in the antibody-drug conjugate of general formula (Pc-L-Y-D) or the pharmaceutically acceptable salt thereof according to any one of the above,

Y is -O-($CR^aR^b$)$_m$-$CR^1R^2$-C(O)-;

$R^a$ and $R^b$ are identical or different and are each independently selected from the group consisting of hydrogen, deuterium, halogen and $C_{1-6}$ alkyl;

$R^1$ is $C_{1-6}$ haloalkyl or $C_{3-6}$ cycloalkyl;

$R^2$ is selected from the group consisting of hydrogen, $C_{1-6}$ haloalkyl and $C_{3-6}$ cycloalkyl; or, $R^1$ and $R^2$, together with the carbon atom to which they are attached, form $C_{3-6}$ cycloalkyl;

m is 0 or 1.

In some embodiments, in the antibody-drug conjugate of general formula (Pc-L-Y-D) or the pharmaceutically acceptable salt thereof according to any one of the above, Y is selected from the group consisting of:

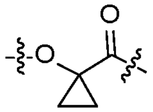, 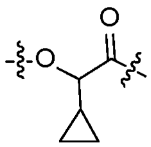, 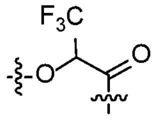, 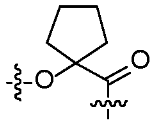, 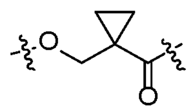, 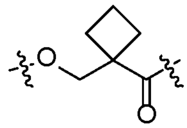,

and

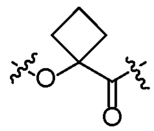;

wherein the O-terminus of Y is connected to the linker unit L.

In some embodiments, in the antibody-drug conjugate of general formula (Pc-L-Y-D) or the pharmaceutically acceptable salt thereof according to any one of the above, the linker unit -L- is $-L^1-L^2-L^3-L^4-$, wherein

$L^1$ is selected from the group consisting of -(succinimidyl-3-yl-N)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-$C_{3-6}$ cycloalkyl and linear heteroalkyl of 1 to 8 chain atoms, and the linear heteroalkyl of 1 to 8 chain atoms comprises 1 to 3 heteroatoms selected from the group consisting of N, O and S, wherein the $C_{1-8}$ alkyl, $C_{1-8}$ alkyl-$C_{3-6}$ cycloalkyl and linear heteroalkyl of 1 to 8 chain atoms are each independently optionally further substituted with one or more substituents selected from the group consisting of halogen, hydroxy, cyano, amino, $C_{1-6}$ alkyl, $C_{1-6}$ haloalkyl, deuterated $C_{1-6}$ alkyl, $C_{1-6}$ alkoxy and $C_{3-6}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5$, $-C(O)NR^5(CH_2)_t-$and a chemical bond, wherein t is an integer from 1 to 6;

$R^3$, $R^4$ and $R^5$ are identical or different and are each independently selected from the group consisting of hydrogen, $C_{1-6}$ alkyl, $C_{1-6}$ haloalkyl, deuterated $C_{1-6}$ alkyl and $C_{1-6}$ hydroxyalkyl;

$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and $C_{1-6}$ hydroxyalkyl.

In some embodiments, in the antibody-drug conjugate of general formula (Pc-L-Y-D) or the pharmaceutically acceptable salt thereof according to any one of the above, the linker unit -L- is $-L^1-L^2-L^3-L^4-$, wherein

$L^1$ is

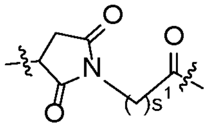,

and $s^1$ is an integer from 2 to 8;

$L^2$ is a chemical bond;

$L^3$ is a tetrapeptide residue;

$L^4$ is $-NR^5(CR^6R^7)t-$, wherein $R^5$, $R^6$ and $R^7$ are identical or different and are each independently hydrogen or $C_{1-6}$ alkyl, and t is 1 or 2;

wherein the $L^1$ terminus is connected to Pc, and the $L^4$ terminus is connected to Y

In some embodiments, in the antibody-drug conjugate of general formula (Pc-L-Y-D) or the pharmaceutically acceptable salt thereof according to any one of the above, $L^3$ is a tetrapeptide residue of GGFG.

In some embodiments, in the antibody-drug conjugate of general formula (Pc-L-Y-D) or the pharmaceutically acceptable salt thereof according to any one of the above, -L- is:

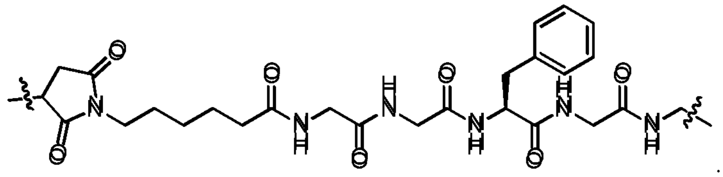

.

In some embodiments, in the antibody-drug conjugate of general formula (Pc-L-Y-D) or the pharmaceutically acceptable salt thereof according to any one of the above, -L-Y- is optionally selected from the group consisting of:

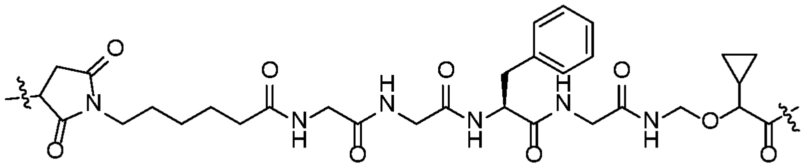

,

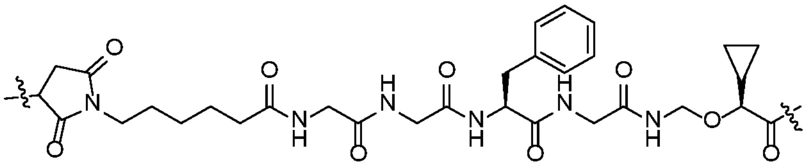

and

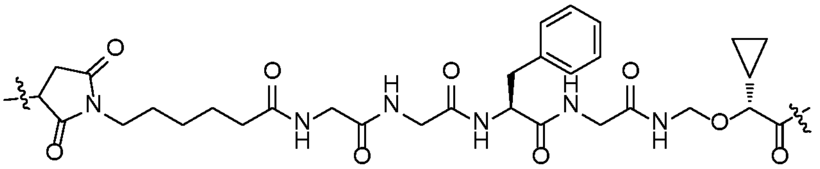

.

In some embodiments, the antibody-drug conjugate of general formula (Pc-L-Y-D) or the pharmaceutically acceptable salt thereof according to any one of the above is an antibody-drug conjugate of general formula (Pc-$L_a$-Y-D) or a pharmaceutically acceptable salt thereof:

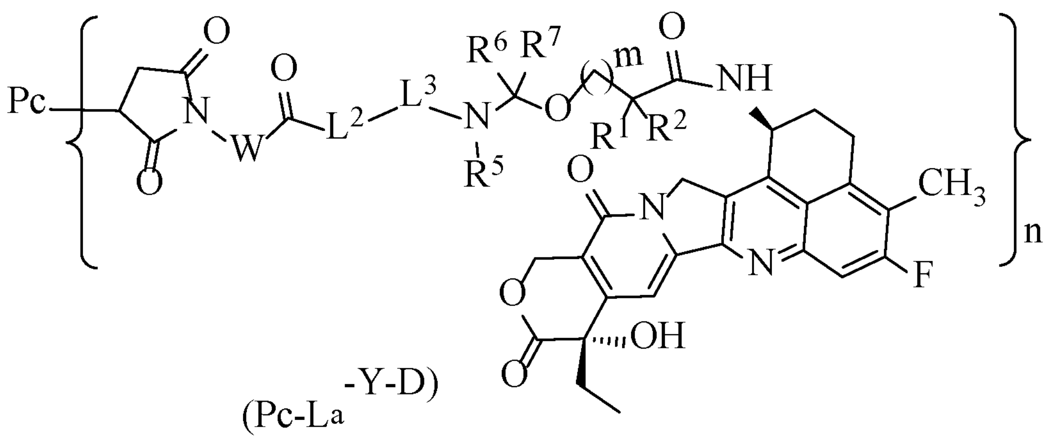

(Pc-$L_a$-Y-D)

wherein,

Pc is the anti-HER3 antibody described above;

m is an integer from 0 to 4; for example, m is selected from the group consisting of 0, 1, 2, 3 and 4;

n is a decimal or an integer from 1 to 10; specifically, n is a decimal or an integer of between 2 and 8 inclusive; more specifically, n is a decimal or an integer from 2 to 7 inclusive; alternatively, n is a decimal or an integer of between 2 and 3, 3 and 4, 4 and 5, 5 and 6, 6 and 7, or 7 and 8, inclusive;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with the carbon atom to which they are attached, form cycloalkyl or heterocyclyl;

W is selected from the group consisting of $C_{1-8}$ alkyl, $C_{1-8}$ alkyl-$C_{3-6}$ cycloalkyl and linear heteroalkyl of 1 to 8 chain atoms, and the heteroalkyl of 1 to 8 chain atoms comprises 1 to 3 heteroatoms selected from the group consisting of N, O and S, wherein the $C_{1-8}$ alkyl, $C_{1-8}$ alkyl-$C_{3-6}$ cycloalkyl and linear heteroalkyl of 1 to 8 chain atoms are each independently optionally further substituted with one or more substituents selected from the group consisting of halogen, hydroxy, cyano, amino, $C_{1-8}$ alkyl, $C_{1-6}$ chloroalkyl, deuterated $C_{1-6}$ alkyl, $C_{1-6}$ alkoxy, and $C_{3-6}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, alkyl, chloroalkyl, deuterated alkyl, alkoxy and cycloalkyl;

$R^5$ is selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

In some embodiments, the antibody-drug conjugate of general formula (Pc-L-Y-D) or the pharmaceutically acceptable salt thereof according to any one of the above is an antibody-drug conjugate of general formula (Pc-$L_a$-Y-D) or a pharmaceutically acceptable salt thereof, wherein

Pc is the anti-HER3 antibody according to any one of the above;

m is an integer from 0 to 4; for example, m is selected from the group consisting of 0, 1, 2, 3 and 4;

n is a decimal or an integer from 1 to 10; specifically, n is a decimal or an integer of between 2 and 8 inclusive; more specifically, n is a decimal or an integer from 2 to 7 inclusive; alternatively, n is a decimal or an integer of between 2 and 3, 3 and 4, 4 and 5, 5 and 6, 6 and 7, or 7 and 8, inclusive;

$R^1$ is selected from the group consisting of halogen, $C_{1-6}$ haloalkyl, deuterated $C_{1-6}$ alkyl, $C_{3-6}$ cycloalkyl, $C_{3-6}$ cycloalkyl $C_{1-6}$ alkyl, $C_{1-6}$ alkoxy $C_{1-6}$ alkyl, heterocyclyl, aryl and heteroaryl; $R^2$ is selected from the group consisting of hydrogen, halogen, $C_{1-6}$ haloalkyl, deuterated $C_{1-6}$ alkyl, $C_{3-6}$ cycloalkyl, $C_{3-6}$ cycloalkyl $C_{1-6}$ alkyl, $C_{1-6}$ alkoxy $C_{1-6}$ alkyl, heterocyclyl, aryl and heteroaryl; or, $R^1$ and $R^2$, together with the carbon atom to which they are attached, form $C_{3-6}$ cycloalkyl or heterocyclyl;

W is selected from the group consisting of $C_{1-8}$ alkyl, $C_{1-8}$ alkyl-$C_{3-6}$ cycloalkyl and linear heteroalkyl of 1 to 8 chain atoms, and the heteroalkyl of 1 to 8 chain atoms comprises 1 to 3 heteroatoms selected from the group consisting of N, O and S, wherein the $C_{1-8}$ alkyl, $C_{1-8}$ alkyl-$C_{3-6}$ cycloalkyl and linear heteroalkyl of 1 to 8 chain atoms are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy, and $C_{3-6}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-6}$ cycloalkyl;

$R^5$ is selected from the group consisting of hydrogen, $C_{1-6}$ alkyl, $C_{1-6}$ haloalkyl, deuterated $C_{1-6}$ alkyl and $C_{1-6}$ hydroxyalkyl;

$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and $C_{1-6}$ hydroxyalkyl;

the heterocyclyl comprises 3 to 6 ring atoms, of which 1 to 3 are heteroatoms selected from the group consisting of nitrogen, oxygen and sulfur.

In some embodiments, in the antibody-drug conjugate of general formula (Pc-L-Y-D) or the pharmaceutically acceptable salt thereof according to any one of the above, the antibody-drug conjugate is:

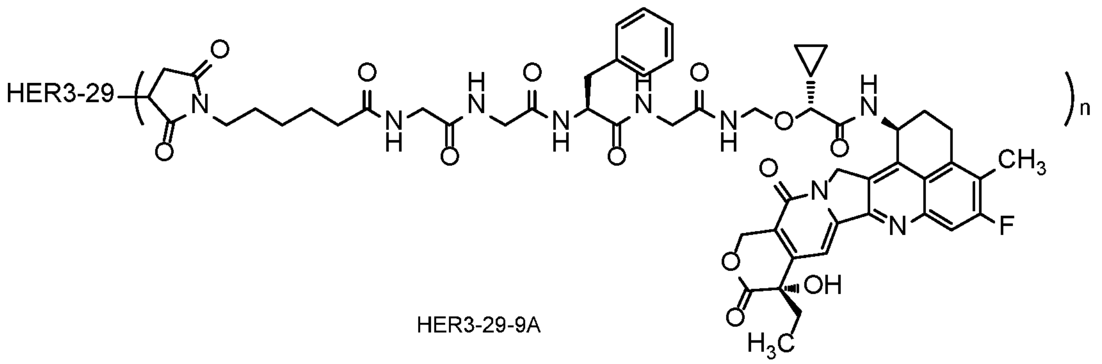

HER3-29-9A

wherein:

n is a decimal or an integer from 1 to 8; specifically, n is a decimal or an integer of between 2 and 8 inclusive; more specifically, n is a decimal or an integer from 2 to 7 inclusive; alternatively, n is a decimal or an integer of between 2 and 3, 3 and 4, 4 and 5, 5 and 6, 6 and 7, or 7 and 8, inclusive;

HER3-29 is an anti-HER3 antibody comprising a heavy chain set forth in SEQ ID NO: 27 and a light chain set forth in SEQ ID NO: 28.

In some embodiments, in the antibody-drug conjugate of general formula (Pc-L-Y-D) or the pharmaceutically acceptable salt thereof according to any one of the above, n is preferably a decimal or an integer from 3 to 8.

In some embodiments, the present disclosure also provides a method for preparing the antibody-drug conjugate of general formula (Pc-$L_a$-Y-D) or the pharmaceutically acceptable salt thereof according to any one of the above comprising the following steps:

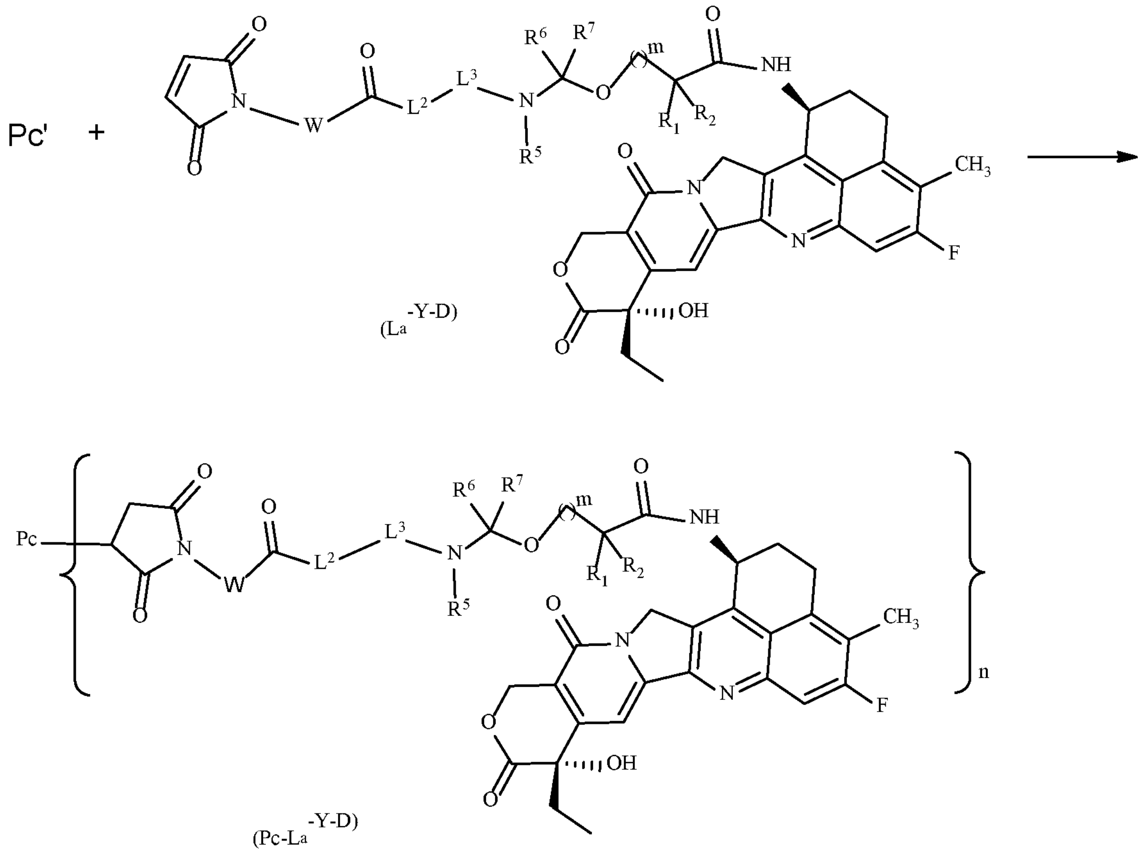

(La-Y-D)

(Pc-La-Y-D)

conducting a coupling reaction of Pc' with a compound of general formula ($L_a$-Y-D) to give a compound of general formula (Pc-$L_a$-Y-D); wherein:

Pc' is obtained by reducing Pc;
n, m, W, $L^2$, $L^3$, $R^1$, $R^2$, $R^5$, $R^6$ and $R^7$ are as defined in any one of the above.

In some embodiments, the present disclosure also provides a pharmaceutical composition comprising the anti-HER3 antibody according to any one of the above, or the nucleic acid molecule according to any one of the above, or the antibody-drug conjugate or the pharmaceutically acceptable salt thereof according to any one of the above, and one or more pharmaceutically acceptable excipients, diluents or carriers.

In some embodiments, the present disclosure also provides use of the anti-HER3 antibody according to any one of the above, or the nucleic acid molecule according to any one of the above, or the antibody-drug conjugate or the pharmaceutically acceptable salt thereof according to any one of the above, or the pharmaceutical composition according to any one of the above, in preparing a medicament for treating a HER3-mediated disease or disorder.

In some embodiments, the present disclosure also provides use of the anti-HER3 antibody according to any one of the above, or the nucleic acid molecule according to any one of the above, or the antibody-drug conjugate or the pharmaceutically acceptable salt thereof according to any one of the above, or the pharmaceutical composition according to any one of the above, in preparing a medicament for treating and/or preventing tumors and cancers, wherein the tumors and cancers are selected from the group consisting of breast cancer, non-small cell lung cancer, gastric cancer, ovarian cancer, prostate cancer, bladder cancer, colorectal cancer, head and neck squamous cell carcinoma and melanoma.

In some embodiments, the present disclosure also provides a kit comprising the anti-HER3 antibody according to any one of the above, or the nucleic acid molecule according to any one of the above, or the antibody-drug conjugate or the pharmaceutically acceptable salt thereof according to any one of the above, or the pharmaceutical composition according to any one of the above.

In some embodiments, the present disclosure also provides a method for preventing or treating a disease or disorder comprising administering to a subject a therapeutically effective amount of the anti-HER3 antibody according to any one of the above, or the nucleic acid molecule according to any one of the above, or the antibody-drug conjugate or the pharmaceutically acceptable salt thereof according to any one of the above, or the pharmaceutical composition according to any one of the above. In some embodiments, the disease or disorder is preferably a tumor, an autoimmune disease, or an infectious disease; in some embodiments, the disease or disorder is a disease or disorder associated with HER3.

In another aspect, the present disclosure provides a pharmaceutical composition comprising the anti-HER3 antibody, the antibody-drug conjugate or the pharmaceutically acceptable salt thereof according to any one of the above, and one or more pharmaceutically acceptable excipients, diluents or carriers. In some embodiments, a unit dose of the pharmaceutical composition comprises 0.1-3000 mg or 1-1000 mg of the anti-HER3 antibody described above or the antibody drug conjugate described above.

In another aspect, the present disclosure provides use of the antibody-drug conjugate or the pharmaceutically acceptable salt thereof or the pharmaceutical composition comprising the same according to any one of the above as a medicament.

In another aspect, the present disclosure provides use of the antibody-drug conjugate or the pharmaceutically acceptable salt thereof or the pharmaceutical composition comprising the same according to any one of the above in preparing a medicament for treating a HER3-mediated disease or disorder; in some embodiments, the HER3-mediated disease or disorder is a cancer with high, moderate or low HER3 expression.

In another aspect, the present disclosure provides use of the antibody-drug conjugate or the pharmaceutically acceptable salt thereof or the pharmaceutical composition comprising the same according to any one of the above in preparing a medicament for treating or preventing cancer; in some embodiments, the tumor and cancer are selected from the group consisting of breast cancer, non-small cell lung cancer, gastric cancer, ovarian cancer, prostate cancer, bladder cancer, colorectal cancer, head and neck squamous cell carcinoma and melanoma.

In another aspect, the present disclosure further relates to a method for treating and/or preventing a tumor comprising administering to a subject in need thereof a therapeutically effective dose of the antibody-drug conjugate or the pharmaceutically acceptable salt thereof or the pharmaceutical composition comprising the same according to any one of the above; in some embodiments, the tumor is a cancer associated with high, moderate or low expression of HER3.

In another aspect, the present disclosure further relates to a method for treating or preventing tumors or cancers comprising administering to a subject in need thereof a therapeutically effective dose of the antibody drug conjugate or the pharmaceutically acceptable salt thereof or the pharmaceutical composition comprising the same according to any one of the above; in some embodiments, the tumors and cancers are selected from the group consisting of breast cancer, non-small cell lung cancer, gastric cancer, ovarian cancer, prostate cancer, bladder cancer, colorectal cancer, head and

neck squamous cell carcinoma and melanoma.

In another aspect, the present disclosure further provides the anti-HER3 antibody or the antibody-drug conjugate thereof according to any one of the above as a medicament, in some embodiments, as a medicament for treating cancers or tumors, more preferably as a medicament for treating HER3-mediated cancer.

The active compound (e.g., the compound or the pharmaceutically acceptable salt thereof according to the present disclosure, or the ligand-drug conjugate or the pharmaceutically acceptable salt thereof according to the present disclosure) may be formulated in a form suitable for administration by any suitable route. The active compound may be in the form of a unit dose, or in the form of a single dose that can be self-administered by a subject. The unit dose of the active compound or composition of the present disclosure may be expressed in the form of a tablet, a capsule, a cachet, a vial, a powder, a granule, a lozenge, a suppository, a powder for reconstitution or a liquid formulation.

The administration dose of the active compound or composition used in the treatment method of the present disclosure will generally vary with the severity of the disease, the weight of the subject, and the relative efficacy of the active compound. However, as a general guide, a suitable unit dose may be 0.1-1000 mg.

The pharmaceutical composition of the present disclosure may comprise, in addition to the active compound, one or more excipients selected from the group consisting of a filler, a diluent, a binder, a wetting agent, a disintegrant, an excipient and the like. Depending on the method of administration, the composition may comprise 0.1 wt.% to 99 wt.% of the active compound.

The HER3 antibody and the antibody drug conjugate provided by the present disclosure have good affinity for cell surface antigens, good endocytosis efficiency and high tumor inhibition efficiency as well as wider drug application windows, and are suitable for clinical drug application.

BRIEF DESCRIPTION OF THE DRAWINGS

FIG. 1: the binding activity of the antibody of the present disclosure and a positive antibody to HER3 protein.

FIG. 2: the binding activity of the antibody of the present disclosure and a positive antibody to MCF7 cells.

FIG. 3: the testing of the cellular endocytic activity of the antibody of the present disclosure and a positive antibody by DT3C.

FIG. 4: the testing of the cellular endocytic activity of the antibody of the present disclosure and a positive antibody by pHrodo.

FIG. 5: the efficacy of the ADC samples of the present disclosure on SW620 xenograft tumors in tumor-bearing nude mice.

## DETAILED DESCRIPTION

### Terms

Unless otherwise defined, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, it is intended to include the formulation of the commercial product under the trade name, and the drug and active drug component of the commercial product under the trade name.

The term "antibody-drug conjugate" (ADC) refers to the linking of an antibody to a biologically active drug. The antibody may be coupled to the drug directly or via a linker unit.

The term "drug loading" refers to the average number of drugs that each antibody-drug conjugate molecule carries, and can also be expressed as a ratio of the number of drugs to the number of antibodies. Drug loading may range from 0 to 12 drugs, illustratively 1, 2, 3, 4, 5, 6, 7, 8, 9 or 10 drugs, linked per antibody; the numerical value may be a decimal or an integer. In certain embodiments, each antibody carries 1 to about 10 drugs; in certain embodiments, each antibody carries about 1 to about 9, 1 to about 8, about 3 to about 7, about 3 to about 6, about 3 to about 5, about 2, about 3, about 4, about 5, about 6, about 7 or about 8 drugs; the numerical value may be a decimal or an integer. Drug loading can be identified by conventional methods, such as UV/visible spectroscopy, mass spectrometry, ELISA assays, and HPLC characterization.

In one embodiment of the present disclosure, the cytotoxic drug is coupled to a sulfhydryl group of the antibody by a linker unit.

The loading of the ligand cytotoxic drug conjugate can be controlled by the following non-limiting methods,

including:

(1) controlling a molar ratio of a linking reagent to a monoclonal antibody,
(2) controlling reaction time and temperature, and
(3) selecting different reaction reagents.

The three-letter and single-letter codes for amino acids used herein are as described in J. biol. chem, 243, p3558 (1968).

The term "antibody" herein is used in the broadest sense and encompasses a variety of antibody structures, including but not limited to monoclonal antibodies, polyclonal antibodies, multispecific antibodies (e.g., bispecific antibodies), full-length antibodies or antigen-binding fragments thereof (also known as antigen-binding moieties) so long as they exhibit the desired antigen-binding activity. A full-length antibody is an immunoglobulin (Ig) that comprises at least two heavy chains and two light chains interconnected by disulfide bonds. The heavy chain constant regions of immunoglobulins differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are classified into $\kappa$ or $\lambda$ chains according to differences in the constant regions. Each of the five classes of Ig may have a $\kappa$ chain or $\lambda$ chain.

In the heavy and light chains of the full-length antibody, the sequences of about 110 amino acids near the N-terminus vary considerably and thus are referred to as variable regions (abbreviated as Fv regions); the remaining amino acid sequences near the C-terminus are relatively stable and thus are referred to as constant regions. Each heavy chain consists of a heavy chain variable region (abbreviated as VH) and a heavy chain constant region (abbreviated as CH). The heavy chain constant region comprises three regions (domains), i.e., CH1, CH2 and CH3. Each light chain consists of a light chain variable region (abbreviated as VL) and a light chain constant region (abbreviated as CL). The heavy chain variable region and the light chain variable region comprise hypervariable regions (also referred to as complementarity determining regions, abbreviated as CDRs or HVRs) and framework regions (abbreviated as FRs) whose sequences are relatively conserved. Each VL and VH consist of 3 CDRs and 4 FRs arranged from the amino terminus to the carboxyl terminus in the following order: FR1-CDR1-FR2-CDR2-FR3-CDR3-FR4. The 3 CDRs of the light chain refer to LCDR1, LCDR2 and LCDR3, and the 3 CDRs of the heavy chain refer to HCDR1, HCDR2 and HCDR3.

The "conventional variant" of the human antibody heavy chain constant region and the human antibody light chain constant region described herein refers to a variant of the heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are, for example, YTE mutation, L234A and/or L235A mutation, or S228P mutation, 265A (e.g., D265A) and/or 297A (e.g., N297A), and/or mutations to obtain a knob-into-hole structure (so that the antibody heavy chain has a combination of knob-Fc and hole-Fc) known in the art. These mutations have been confirmed to make the antibody have new properties, but do not change the function of the antibody variable region.

"Human antibody" (HuMAb), "human-derived antibody", "fully human antibody" and "completely human antibody" herein are used interchangeably and have amino acid sequences corresponding to those of antibodies produced by humans or human cells, or derived from non-human sources using repertoires of human antibodies or other human antibody coding sequences. The definition of such a human antibody specifically excludes humanized antibodies comprising non-human antigen-binding residues.

The term "antigen-binding fragment" or "functional fragment" or "antigen-binding moiety" refers to one or more fragments of an intact antibody that retain the ability to specifically bind to an antigen. A fragment of a full-length antibody can be used to perform the antigen-binding function of the antibody. Illustratively, examples of the binding fragment included in the term "antigen-binding fragment" include (i) a Fab fragment, a monovalent fragment consisting of VL, VH, CL and CH1 domains; (ii) an $F(ab')_2$ fragment, a bivalent fragment comprising two Fab fragments linked by a disulfide bridge in the hinge region; (iii) an Fd fragment, consisting of VH and CH1 domains; (iv) an Fv fragment, consisting of VH and VL domains of one arm of the antibody; (V) a dsFv, a stable antigen-binding fragment formed by VH and VL via interchain disulfide bonds therebetween; (vi) a diabody, a bispecific antibody and a multi-specific antibody, comprising such fragments as an scFv, a dsFv and a Fab. Furthermore, although the two domains of the Fv fragment, VL and VH, are encoded by separate genes, these two domains can be linked by a recombinant method using an artificial peptide linker that enables them to be formed as a single protein chain, wherein the VL and VH pair to form a monovalent molecule, referred to as single-chain Fv (scFv) (see, e.g., Bird et al. (1988) Science 242: 423-426; and Huston et al. (1988) Proc. Natl. Acad. Sci USA 85:5879-5883). Such single-chain antibodies are also included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzymatic or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

The term "amino acid difference" or "amino acid mutation" refers to the presence of amino acid changes or mutations in the variant protein or polypeptide compared with the original protein or polypeptide, including occurrence of 1, 2, 3 or more amino acid insertions, deletions or substitutions on the basis of the original protein or polypeptide. The term "antibody framework region" or "FR" refers to a portion of a variable domain VL or VH, which serves as a framework for the antigen-binding loops (CDRs) of the variable domain. It is essentially a variable domain without CDRs.

The term "complementarity-determining region", "CDR" or "hypervariable region" refers to one of the 6 hypervariable regions within the variable domain of an antibody which primarily contribute to antigen binding. Generally, there are three CDRs (HCDR1, HCDR2 and HCDR3) in each heavy chain variable region and three CDRs (LCDR1, LCDR2 and LCDR3) in each light chain variable region. The amino acid sequence boundaries of the CDRs can be determined using any of a variety of well-known schemes, including the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001) and the ImMunoGenTics (IMGT) numbering scheme (see Lefranc, M.P., Dev. Comp. Immunol., 27, 55-77(2003)), and the like. For example, for the classical format, according to the Kabat scheme, the CDR amino acid residues in the heavy chain variable domain (VH) are numbered 31-35 (HCDR1), 50-65 (HCDR2) and 95-102 (HCDR3); the CDR amino acid residues in the light chain variable domain (VL) are numbered 24-34 (LCDR1), 50-56 (LCDR2) and 89-97 (LCDR3). According to the Chothia scheme, the CDR amino acids in VH are numbered 26-32 (HCDR1), 52-56 (HCDR2) and 95-102 (HCDR3); and the amino acid residues in VL are numbered 24-34 (LCDR1), 50-56 (LCDR2) and 89-97 (LCDR3). According to the IMGT scheme, the CDR amino acid residues in VH are roughly numbered 27-38 (HCDR1), 56-65 (HCDR2) and 105-117 (HCDR3), and the CDR amino acid residues in VL are roughly numbered 27-38 (LCDR1), 56-65 (LCDR2) and 105-117 (LCDR3). According to the AbM scheme, the CDR amino acids in VH are numbered 26-35 (HCDR1), 50-58 (HCDR2) and 95-102 (HCDR3); and the amino acid residues in VL are numbered 24-34 (LCDR1), 50-56 (LCDR2) and 89-97 (LCDR3).

The term "epitope" or "antigenic determinant" refers to a site on an antigen (e.g., a specific site on a HER3 molecule) to which an antibody binds. Epitopes generally comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively bind to" and "specifically bind to" refer to the binding of an antibody or antigen-binding fragment to an epitope on a predetermined antigen. Generally, the antibody or antigen-binding fragment binds with an affinity (KD) of less than about $10^{-8}$ M, e.g., less than about $10^{-9}$ M, $10^{-10}$ M, $10^{-11}$ M, $10^{-12}$ M, or less.

The term "compete", when used in a case where antigen-binding proteins (e.g., neutralizing antigen-binding proteins or neutralizing antibodies) compete for the same epitope, refers to the competition between the antigen-binding proteins, which is determined by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen (e.g., HER3 antigen or a fragment thereof). Numerous types of competitive binding assays are available for determining whether an antigen-binding protein competes with another, such as: solid phase direct or indirect radioimmunoassay (RIA), solid phase direct or indirect enzyme immunoassay (EIA), and sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with I-125 label (see, e.g., Morel et al., 1988, Molec. Immunol. 25: 7-15); solid phase direct biotin-avidin EIA (see, e.g., Cheung, et al., 1990, Virology 176: 546-552) and direct labeled RIA (Moldenhauer et al., 1990, Scand. J. Immunol. 32: 77-82). Generally, the assay relates to use of a purified antigen binding to a solid surface or a cell bearing any of an unlabeled assayed antigen-binding protein and a labeled reference antigen-binding protein. Competitive inhibition is determined by measuring the amount of label bound to the solid surface or the cell in the presence of the assayed antigen-binding protein. Generally, the assayed antigen-binding protein exists in an excessive amount. Antigen binding proteins identified by competitive assays (competing antigen binding proteins) include an antigen binding protein binding to the same epitope as a reference antigen binding protein and an antigen binding protein binding to a proximal epitope sufficiently close to the binding epitope of the reference antigen binding protein, wherein the two epitopes sterically hinder binding from occurring. Other detailed information regarding the method for assaying competitive binding is provided in the examples herein. Generally, when the competitive antigen-binding protein exists in an excessive amount, the specific binding of the reference antigen-binding protein to the common antigen will be inhibited (e.g., reduced) by at least 40%-45%, 45%-50%, 50%-55%, 55%-60%, 60%-65%, 65%-70%, 70%-75% or 75% or more. In certain instances, the binding is inhibited by at least 80%-85%, 85%-90%, 90%-95%, 95%-97% or 97% or more.

The term "nucleic acid molecule" used herein refers to a DNA molecule or an RNA molecule. The nucleic acid molecule may be single-stranded or double-stranded, and is preferably a double-stranded DNA, a single-stranded mRNA or a modified mRNA. A nucleic acid is "operably linked" when it is placed into a functional relationship with another nucleic acid sequence. For example, a promoter or enhancer is operably linked to a coding sequence if it affects the transcription of the coding sequence.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the skill in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

"Antibody-dependent cell-mediated cytotoxicity" or "ADCC" refers to a cell-mediated response in which nonspecific cytotoxic cells expressing FcRs (e.g., natural killer (NK) cells, neutrophils, and macrophages) recognize bound antibody on a target cell, resulting in lysis of the target cell. Primary cells and NK cells that regulate ADCC express FcγRIII only, whereas monocytes express FcγRI, FcγRII and FcγRIII. *In vivo* and *in vitro* ADCC assays may be performed to assess ADCC activity of a molecule of interest, such as those described by Clynes et al. (PNAS USA 95: 652-656 (1998)), and in US Patent Nos. US5500362 and US5821337, and the like.

"Antibody-dependent cellular phagocytosis" or "ADCP" refers to the mechanism by which antibody-coated target cells or virions are eliminated by internalization of phagocytic cells (e.g., macrophages, neutrophils, and dendritic cells). Internalized antibody-coated target cells or virions are contained in vesicles called phagosomes, which are subsequently fused to one or more lysosomes to form phagolysosomes. ADCP can be assessed by an *in vitro* cytotoxicity assay using macrophages as effector cells and videomicroscopy (e.g., van Bij et al., Journal of Hepatology Vol. 53, No. 4, October 2010, p677-685). "Complement-dependent cytotoxicity" or "CDC" refers to cytotoxicity in which complement is involved, i.e., a lytic effect on the target cell by a membrane attack complex that is formed by the activation of the classical pathway of complement after binding of an antibody to the corresponding antigen on a cell or virion to form a complex. CDC can be assessed by an *in vitro* assay (e.g., a CDC assay using normal human serum as a source of complement) or in a series of C1q concentrations. A decrease in CDC activity (e.g., a decrease in CDC activity due to the introduction of a second mutation in a polypeptide or antibody) can be determined by comparing the CDC activity of the polypeptide or antibody to the CDC activity of a parent polypeptide or antibody that does not have the second mutation in the same assay. An assay such as that described by Romeuf et al (Romeuf et al., Br J Haematol. 2008 Mar; 140(6): 635-43) can be performed to assess the ability of an antibody to induce CDC.

The antibody or the antibody fragment described herein may be coupled to the effector molecule by any means. For example, the antibody or the antibody fragment may be chemically or recombinantly attached to the cytotoxic drug. Chemical means for preparing fusions or conjugates are known in the art and can be used to prepare immunoconjugates. The method for conjugating the antibody or the antibody fragment and the drug must be capable of linking the antibody to the cytotoxic drug without interfering with the ability of the antibody or the antibody fragment to bind to the target molecule.

In one embodiment, both the antibody and cytotoxic drug are proteins and can be coupled using techniques well known in the art. There are hundreds of cross-linking agents disclosed in the art that can conjugate two proteins. The cross-linking agent is generally selected based on reactive functional groups available or inserted on the antibody or cytotoxic drug. Alternatively, if no reactive groups are present, a photo-activatable crosslinking agent may be used. In some cases, it may be desirable to include a spacer between the antibody and the cytotoxic drug. Cross-linking agents known in the art include homobifunctional agents: glutaraldehyde, dimethyl adipimidate and bis(diazobenzidine), and heterobifunctional agents: *m*-maleimidobenzoyl-N-hydroxysuccinimide and sulfo-*m*-maleimidobenzoyl-*N*-hydroxysuccinimide.

Cross-linking agents that can be used to conjugate an effector molecule to an antibody fragment include, for example, TPCH (S-(2-thiopyridyl)-L-cysteine hydrazide) and TPMPH (S-(2-thiopyridyl)sulfhydryl-propionhydrazide). TPCH and TPMPH react on the carbohydrate moiety of the glycoprotein that had previously been oxidized by mild periodate treatment, thereby forming a hydrazone bond between the hydrazide moiety of the crosslinking agent and the aldehyde generated by periodate. The heterobifunctional cross-linking agents GNMS (N-(γ-maleimidobutyryloxy)-succinimide) and SMCC (succinimidyl 4-(*N*-maleimido-methyl)cyclohexane) are reacted with a primary amine, thereby introducing a maleimido group onto the component. This maleimido group may then react with a sulfhydryl group on another component which may be introduced by a cross-linking agent, thereby forming a stable thioether bond between the components. If

steric hindrance between the components interferes with the activity of either component, a cross-linking agent may be used to introduce a long spacer between the components, such as N-succinimidyl 3-(2-pyridyldithio)propionate (SPDP). Thus, there are many suitable cross-linking agents that may be used and selected individually depending on their effect on the yield of the optimal immunoconjugate.

The term "expression vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector, wherein additional DNA segments may be ligated into the viral genome. The vectors disclosed herein are capable of autonomous replication in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

Methods for producing and purifying antibodies and antigen-binding fragments are well known in the art, for example, those described in chapters 5-8 and 15 of "Antibodies: A Laboratory Manual", Cold Spring Harbor Press. The antibody or the antigen-binding fragment described herein is genetically engineered to contain one or more additional human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell lines), 293 and NS0 cells. In some embodiments, the host cells in the present disclosure do not include cells from human embryos.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Stable clones are obtained by expression of the antibody that specifically binds to human HER3. Positive clones are expanded in a serum-free medium of a bioreactor to produce antibodies. The culture with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Conservative modification" or "conservative replacement or substitution" refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity. Exemplary conservative substitutions are as follows:

| Original residue | Conservative substitution |
|---|---|
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |

(continued)

| Original residue | Conservative substitution |
|---|---|
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

"Exogenous" refers to substances produced outside organisms, cells or human bodies according to circumstances. "Endogenous" refers to substances produced inside cells, organisms or human bodies according to circumstances.

"Homology" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. Generally, two sequences, when aligned, are compared to give the maximum percent homology. For example, the comparison may be made by the BLAST algorithm, wherein the parameters of the algorithm are selected to give the maximum match between the reference sequences over the entire length of each sequence. The following references relate to the BLAST algorithm often used for sequence analysis: the BLAST algorithms: Altschul, S.F. et al., (1990) J. Mol. Biol., 215: 403-410; Gish, W, et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; Zhang, J. et al., (1997) Genome Res., 7: 649-656. Other conventional BLAST algorithms, such as one provided by NCBI BLAST, are also well known to those skilled in the art.

As used herein, the expressions "cell", "cell line" and "cell culture" are used interchangeably, and all such designations include their progenies. Therefore, the words "transformant" and "transformed cell" include primary test cells and cultures derived therefrom, regardless of the number of transfers. It should also be understood that all progenies may not be precisely identical in DNA content due to deliberate or unintentional mutations. Mutant progeny with the same function or biological activity as screened in the original transformed cells is included. When referring to different designations, they will become clear through the context.

"Polymerase chain reaction" or "PCR" used herein refers to a procedure or technique in which a trace amount of a specific moiety of nucleic acid, RNA and/or DNA is amplified as described in, for example, US Patent No. 4,683,195. Generally speaking, it is necessary to obtain sequence information from the end or outside of the target region, so that oligonucleotide primers can be designed; these primers are identical or similar in terms of sequence to the corresponding strand of the template to be amplified. The 5'-terminal nucleotide of 2 primers may coincide with the end of the material to be amplified. PCR can be used to amplify specific RNA sequences, specific DNA sequences from total genomic DNA and cDNA sequences transcribed from total cellular RNA, phage, plasmid sequences, or the like. See generally Mullis, et al., (1987) Cold Spring Harbor Symp. Quant. Biol. 51: 263; Erlich ed. (1989) PCR TECHNOLOGY (Stockton Press, N.Y). The PCR used herein is considered to be an example, but not the only one, of a nucleic acid polymerase reaction method for amplifying a nucleic acid test sample, and the method comprises using known nucleic acids as primers and nucleic acid polymerases to amplify or produce a specific moiety of the nucleic acid.

"Isolated" refers to a purified state, and in this case means that the designated molecule is substantially free

of other biomolecules, such as nucleic acids, proteins, lipids, carbohydrates, or other materials (such as cell debris and growth medium). Generally, the term "isolated" does not mean the complete absence of such substances or the absence of water, buffers or salts, unless they are present in amounts that will significantly interfere with the experimental or therapeutic use of the compounds described herein.

The term "drug" refers to a chemical substance that can alter or ascertain an organism's physiology and pathological state and can be used for the prevention, diagnosis and treatment of diseases. The drug includes a cytotoxic drug. There is no clear boundary between a drug and a toxic substance. The toxic substance refers to a chemical substance that has a toxic effect on organisms and can cause damage to human health even in small doses. Any drug in large doses may induce toxic responses.

The cytotoxic drug refers to a substance that inhibits or prevents cell functions and/or causes cell death or cell destruction. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic drugs, antibiotics and nucleolytic enzymes.

In some embodiments, the toxins may be small molecule toxins from bacteria, fungi, plants or animals and derivatives thereof, including camptothecin derivatives (such as exatecan, and maytansinoids and derivatives thereof (CN101573384) (such as DM1, DM3, DM4, and auristatin F (AF) and derivatives thereof (such as MMAF, MMAE, 3024 (WO 2016/127790 A1, compound 7)))), diphtheria toxin, exotoxin, ricin A chain, abrin A chain, modeccin, α-sarcin, *Aleutites fordii* toxic protein, dianthin toxic protein, *Phytolaca americana* toxic protein (PAPI, PAPII and PAP-S), *Momordica charantia* inhibitor, curcin, crotin, sapaonaria officinalis inhibitor, gelonin, mitogellin, restrictocin, phenomycin, enomycin and trichothecenes.

The term "chemotherapeutic agent" refers to a chemical compound that can be used to treat tumors. The definition also includes anti-hormonal agents that act to modulate, reduce, block or inhibit the effects of hormones that can promote cancer growth, and are often in the form of systematic or systemic treatment. They may themselves be hormones. Examples of chemotherapeutic agents include alkylating agents such as thiotepa and cyclosphamide (CYTOXAN™); alkylsulfonates such as busulfan, improsulfan and piposulfan; aziridine such as benaodopa, carboquone, meturedopa and uredopa; aziridine and melamineamine including altretamine, triethylenemelamine, triethylenephosphoramide, triethylenethiophosphoramide and trimethylolomelamine; nitrogen mustards such as chlorambucil, chlornaphazine, cholophosphamide, estramustine, ifosfamide, mechlorethamine and nitromin hydrochloride; melphalan, novembichin, phenesterine, prednimustine, trofosfamide, and uracil mustard; nitrosureas such as carmustine, chlorozotocin, fotemustine, lomustine, nimustine, and ranimustine; antibiotics such as aclacinomycin, actinomycin, authramycin, azaserine, bleomycin, cactinomycin, calicheamicin, carabicin, chromomycin, carzinophilin, chromomycin, actinomycin D, daunorubicin, detorubicin, 6-diazo-5-oxo-L-norleucine, doxorubicin, epirubicin, esorubicin, idarubicin, marcellomycin, mitomycin, mycophenolic acid, nogalamycin, olivomycin, peplomycin, potfiromycin, puromycin, quelamycin, rodorubicin, and streptonigrin; streptozocin, tuberculocidin, ubenimex, zinostatin, and zorubicin; antimetabolites such as methotrexate and 5-fluorouracil (5-FU); folic acid analogs such as denopterin, methotrexate, pteropterin, and trimetrexate; pterine analogs such as fludarabine, 6-mercaptopterin, thiopterin and thioguanterin; pyrimidine analogs such as ancitabine, azacitidine, 6-azauridine, carmofur, cytarabine, dideoxyuridine, doxitluridine, enocitabine, floxuridine, and 5-FU; androgens such as calusterone, dromostanolong propionate, epitiostanol, mepitiostane, and testolactone; antiadrenergics such as aminoglutethimide, mitotane, and trilostane; folic acid supplements such as frolinic acid; acetogluconolactone; aldophosphamideglycoside; aminolevulinic acid; amsacrine; bestrabucil; biasntrene; edatraxate; defofamine; colchicine; diaziquone; elfomithine; elliptinium acetate; etoglucid; gallium nitrate; hydroxyurea; lentinan; lonidamine; mitoguazone; mitoxantrone; mopidamol; nitracrine; pintostatin; phenamet; pirarubicin; podophyllinic acid; 2-ethylhydrazide; procarbazine; PSK®; razoxane; sizofiran; spirogermanium; tenuazonic acid; triaziquone; 2,2',2"-trichlorrotriethylamine; uretha; vindesine; dacarbazine; mannomustine; mitobronitol; mitolactol; pipobroman; gacytosine; arabinoside ("Ara-C"); cyclophosphamide; thiotepa; taxane such as paclitaxel (TAXOL®, Bristol-Myers Squibb Oncology, Princeton, NJ) and docetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunorubicin; aminopterin; xeloda, and ibandronate; CPT-11; topoisomerase inhibitor RFS2000; difluoromethylomithine (DMFO); retinoic acid; esperamicins; capecitabine; and pharmaceutically acceptable salts, acids or derivatives of any one of the above substances. The definition also includes anti-hormonal agents that can modulate or inhibit the effect of hormones on tumors, such as anti-estrogen agents including tamoxifen, raloxifene, the aromatase inhibitor 4(5)-imidazole, 4-hydroxytamoxifene, trioxifene, keoxifene, LY117018, onapristone, and toremifene (Fareston); and anti-androgen agents such as flutamide, nilutamide, bicalutamide, leuprolide and goserelin; and pharmaceutically acceptable salts, acids or derivatives of any one of the above substances.

The term "alkyl" refers to a saturated aliphatic hydrocarbon group which is a linear or branched group containing

1 to 20 carbon atoms, preferably alkyl containing 1 to 12 (e.g., 1, 2, 3, 4, 5, 6, 7, 8, 9, 10, 11 and 12) carbon atoms, and more preferably alkyl containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various side-chain isomers thereof, and the like. More preferred is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl may be substituted or unsubstituted. When substituted, the substituent may be substituted at any accessible connection site, and the substituent is preferably one or more substituents independently optionally selected from the group consisting of hydrogen, deuterium, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroalkyl" refers to alkyl containing one or more heteroatoms selected from the group consisting of N, O and S, wherein the alkyl is as defined above.

The term "alkylene" refers to a saturated linear or branched aliphatic hydrocarbon group having 2 residues derived by removal of two hydrogen atoms from the same carbon atom or two different carbon atoms of the parent alkane, which is a linear or branched group containing 1 to 20 carbon atoms, preferably an alkylene group containing 1 to 12 (e.g., 1, 2, 3, 4, 5, 6, 7, 8, 9, 10, 11 and 12) carbon atoms, more preferably an alkylene group containing 1 to 8 carbon atoms, and most preferably an alkylene group containing 1 to 6 carbon atoms. Non-limiting examples of alkylene include, but are not limited to, methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), and the like. The alkylene may be substituted or unsubstituted. When substituted, the substituent may be substituted at any accessible connection site, and the substituent is preferably one or more substituents independently optionally selected from the group consisting of alkyl, alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkenyl" refers to an alkyl compound containing a carbon-carbon double bond in the molecule, wherein the alkyl is as defined above. The alkenyl may be substituted or unsubstituted, and when it is substituted, the substituent is preferably one or more groups independently selected from one or more substituents of hydrogen, alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound containing a carbon-carbon triple bond in the molecule, wherein the alkyl is as defined above. The alkynyl may be substituted or unsubstituted, and when it is substituted, the substituent is preferably one or more groups independently selected from one or more substituents of hydrogen, alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 8 (e.g., 3, 4, 5, 6, 7 and 8) carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro cycloalkyl, fused cycloalkyl, and bridged cycloalkyl. The term "spiro cycloalkyl" refers to a 5- to 20-membered polycyclic group in which monocyclic rings share one carbon atom (referred to as the spiro atom). It may contain one or more double bonds, but none of the rings has a fully conjugated $\pi$-electron system. It is preferably 6- to 14-membered, and more preferably 7- to 10-membered (e.g., 7-membered, 8-membered, 9-membered or 10-membered). According to the number of the spiro atoms shared among the rings, the spiro cycloalkyl may be monospiro cycloalkyl, bispiro cycloalkyl or polyspiro cycloalkyl, preferably monospiro cycloalkyl and bispiro cycloalkyl, and more preferably 4-membered/4-membered, 4-membered/5-membered, 4-membered/6-membered, 5-membered/5-membered or 5-membered/6-membered monospiro cycloalkyl. Non-limiting examples of spiro cycloalkyl include:

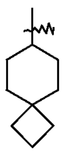 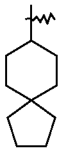 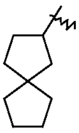 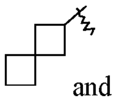 and 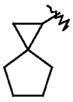 .

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of them has a fully conjugated π-electron system. It is preferably 6- to 14-membered, and more preferably 7- to 10-membered (e.g., 7-membered, 8-membered, 9-membered or 10-membered). According to the number of the formed rings, the fused cycloalkyl may be bicyclic, tricyclic, tetracyclic or polycyclic fused cycloalkyl, preferably bicyclic or tricyclic fused cycloalkyl, and more preferably 5-membered/5-membered or 5-membered/6-membered bicyclic fused heterocyclyl. Non-limiting examples of fused cycloalkyl include:

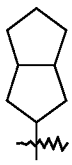 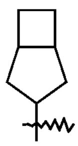 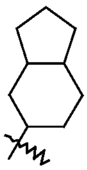 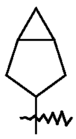 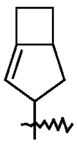 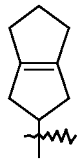 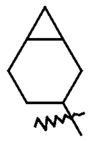 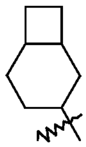 and 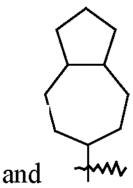 .

The term "bridged cycloalkyl" refers to a 5- to 20-membered all-carbon polycyclic group in which any two rings share two carbon atoms that are not directly connected to each other. It may contain one or more double bonds, but none of the rings has a fully conjugated π-electron system. It is preferably 6- to 14-membered, and more preferably 7- to 10-membered (e.g., 7-membered, 8-membered, 9-membered or 10-membered). According to the number of the formed rings, the bridged cycloalkyl may be bicyclic, tricyclic, tetracyclic or polycyclic, preferably bicyclic, tricyclic or tetracyclic, and more preferably bicyclic or tricyclic. Non-limiting examples of bridged cycloalkyl include:

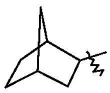 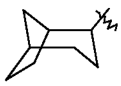 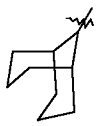 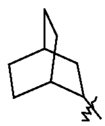 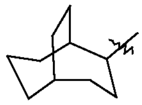

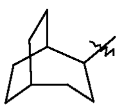 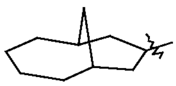 and 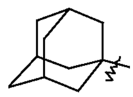 .

The cycloalkyl ring includes those in which the cycloalkyl described above (including monocyclic, spiro, fused and bridged rings) is fused to an aryl, heteroaryl or heterocycloalkyl ring, wherein the ring linked to the parent structure is cycloalkyl. Non-limiting examples include indanyl, tetrahydronaphthyl, benzocycloheptanyl, and the like, preferably benzocyclopentyl or tetrahydronaphthyl.

The cycloalkyl may be substituted or unsubstituted. When substituted, the substituent may be substituted at any accessible connection site, and the substituent is preferably one or more substituents independently optionally selected from the group consisting of hydrogen, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl. The term "alkoxy" refers to -O-(alkyl) and -O-(unsubstituted cycloalkyl), wherein the alkyl and cycloalkyl are as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more groups independently selected from the group consisting of hydrogen, deuterium, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, sulfur, S(O) and $S(O)_2$, excluding a cyclic portion of -O-O-, -O-S- or -S-S-, and the

other ring atoms are carbon atoms. It preferably contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably 3 to 8 (e.g., 3, 4, 5, 6, 7 and 8) ring atoms, of which 1 to 3 (e.g., 1, 2 and 3) are heteroatoms; more preferably 3 to 6 ring atoms, of which 1 to 3 are heteroatoms; most preferably 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more of the ring atoms are heteroatoms selected from the group consisting of nitrogen, oxygen, sulfur, S(O) and $S(O)_2$, and the other ring atoms are carbon atoms. It may contain one or more double bonds, but none of the rings has a fully conjugated π-electron system. It is preferably 6- to 14-membered, and more preferably 7- to 10-membered (e.g., 7-membered, 8-membered, 9-membered or 10-membered). According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. Non-limiting examples of spiro heterocyclyl include:

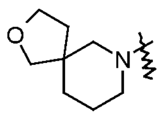 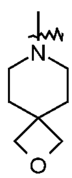 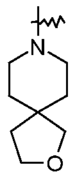 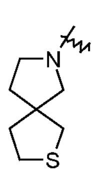 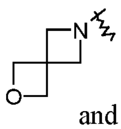 and 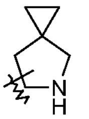 .

The term "fused heterocyclyl" refers to a 5- to 20-membered polycyclic heterocyclyl group in which each ring shares a pair of adj acent atoms with the other rings in the system, wherein one or more rings may contain one or more double bonds, but none of them has a fully conjugated π-electron system, wherein one or more of the ring atoms are heteroatoms selected from the group consisting of nitrogen, oxygen, sulfur, S(O) and $S(O)_2$, and the other ring atoms are carbon atoms. It is preferably 6- to 14-membered, and more preferably 7- to 10-membered (e.g., 7-membered, 8-membered, 9-membered or 10-membered). According to the number of the formed rings, the fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. Non-limiting examples of fused heterocyclyl include:

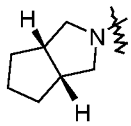 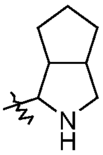 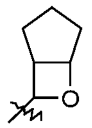 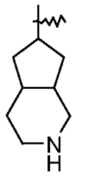 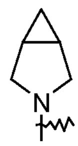 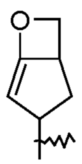 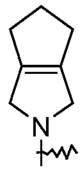 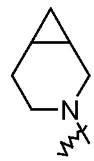

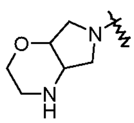 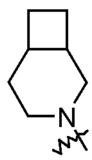 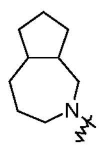 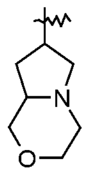 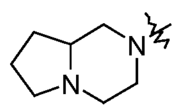 and 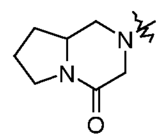 .

The term "bridged heterocyclyl" refers to a 5- to 14-membered polycyclic heterocyclyl group in which any two rings share two atoms that are not directly linked to each other. It may contain one or more double bonds, but none of the rings has a fully conjugated π-electron system, wherein one or more of the ring atoms are heteroatoms selected from the group consisting of nitrogen, oxygen, sulfur, S(O) and $S(O)_2$, and the other ring atoms are carbon atoms. It is preferably 6- to 14-membered, and more preferably 7- to 10-membered (e.g., 7-membered, 8-membered, 9-membered or 10-membered). According to the number of the formed rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

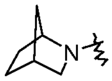 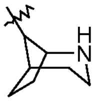 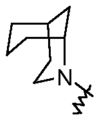 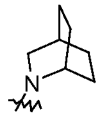

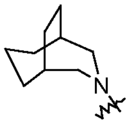 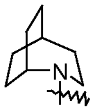 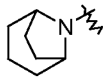 and 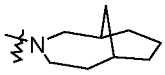

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring linked to the parent structure is heterocyclyl; its non-limiting examples include:

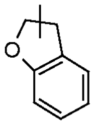 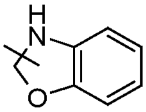 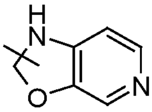, 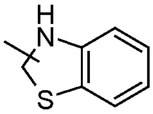, etc.

The heterocyclyl may be substituted or unsubstituted. When substituted, the substituent may be substituted at any accessible connection site, and the substituent is preferably one or more substituents independently optionally selected from the group consisting of hydrogen, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl. The term "aryl" refers to a 6- to 14-membered, preferably 6- to 10-membered carbon monocyclic or fused polycyclic (in which the rings share a pair of adjacent carbon atoms) group having a conjugated π-electron system, such as phenyl and naphthyl. The aryl ring includes those in which the aryl ring described above is fused to a heteroaryl, heterocyclyl or cycloalkyl ring, wherein the ring connected to the parent structure is the aryl ring; its non-limiting examples include:

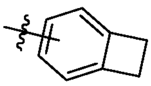 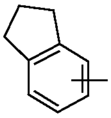 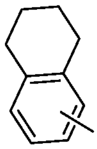 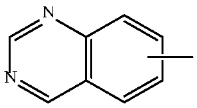 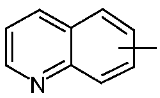 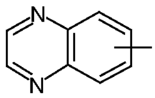 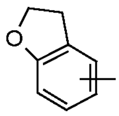

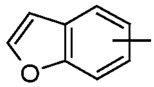 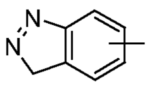 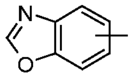 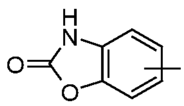 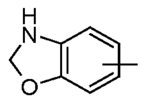 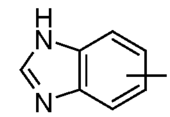

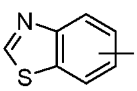 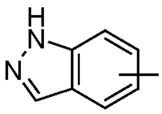 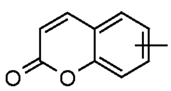 and 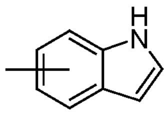.

The aryl may be substituted or unsubstituted. When substituted, the substituent may be substituted at any accessible connection site, and the substituent is preferably one or more substituents independently optionally selected from the group consisting of hydrogen, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered (e.g., 5-membered, 6-membered, 7-membered, 8-membered, 9-membered or 10-membered) and more preferably 5-membered or 6-membered, e.g., furyl, thienyl, pyridinyl, pyrrolyl, N-alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring linked to the parent

structure is the heteroaryl ring; its non-limiting examples include:

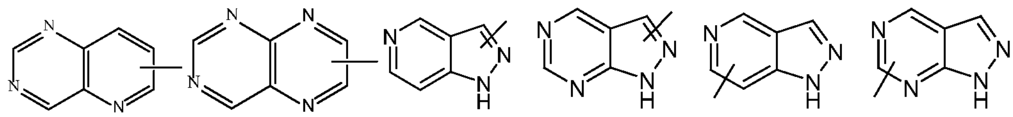

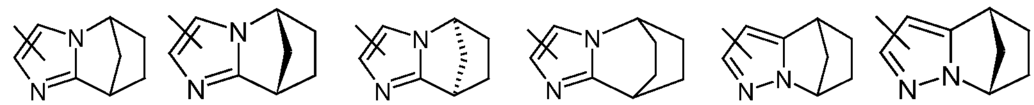

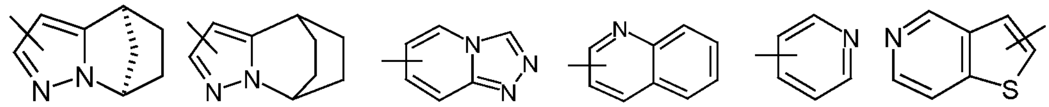

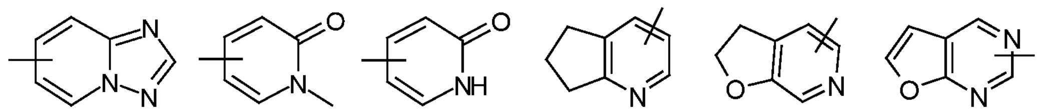

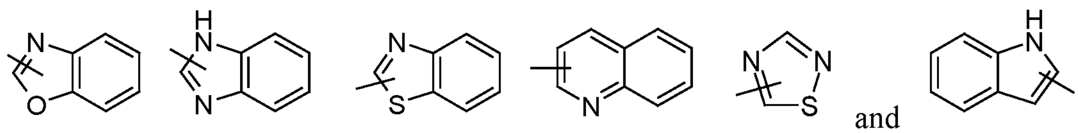

and

The heteroaryl may be substituted or unsubstituted. When substituted, the substituent may be substituted at any accessible connection site, and the substituent is preferably one or more substituents independently optionally selected from the group consisting of hydrogen, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl. The term "amino protecting group" refers to a group that can be easily removed and is intended to protect an amino group from being changed when a reaction is conducted elsewhere in the molecule. Non-limiting examples include (trimethylsilyl)ethoxymethyl, tetrahydropyranyl, tert-butoxycarbonyl, acetyl, benzyl, allyl, p-methoxybenzyl, and the like. These groups may be optionally substituted with 1-3 substituents selected from the group consisting of halogen, alkoxy and nitro.

The term "hydroxy protecting group" is a suitable group known in the art for protecting hydroxy. See the hydroxy protecting groups in the literature ("Protective Groups in Organic Synthesis", 5th Ed. T.W.Greene & P.G.M.Wuts). By way of example, preferably, the hydroxy protecting group may be ($C_{1-10}$ alkyl or aryl)$_3$silyl, e.g., triethylsilyl, triisopropylsilyl, *tert*-butyldimethylsilyl or *tert*-butyldiphenylsilyl; $C_{1-10}$ alkyl or substituted alkyl, preferably alkoxy or aryl-substituted alkyl, more preferably $C_{1-6}$ alkoxysubstituted $C_{1-6}$ alkyl or phenyl-substituted $C_{1-6}$ alkyl, and most preferably $C_{1-4}$ alkoxysubstituted $C_{1-4}$ alkyl, e.g., methyl, *tert*-butyl, allyl, benzyl, methoxymethyl (MOM), ethoxyethyl or 2-tetrahydropyranyl (THP); ($C_{1-10}$ alkyl or aryl)acyl, e.g., formyl, acetyl, benzoyl or *p*-nitrobenzoyl; ($C_{1-6}$ alkyl or $C_{6-10}$ aryl)sulfonyl; or ($C_{1-6}$ alkoxy or $C_{6-10}$ aryloxy)carbonyl.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to an alkyl group substituted with hydroxy, wherein the alkyl is defined as above.

The term "halogen" refers to fluorine, chlorine, bromine or iodine.

The term "hydroxy" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

The compounds disclosed herein include isotopic derivatives thereof. The term "isotopic derivative" refers to compounds that differ in structure only by having one or more enriched isotopic atoms. For example, compounds with the structure of the present disclosure having "deuterium" or "tritium" in place of hydrogen, or $^{18}$F-fluorine labeling ($^{18}$F isotope) in place of fluorine, or $^{11}$C-, $^{13}$C- or $^{14}$C-enriched carbon ($^{11}$C-, $^{13}$C- or $^{14}$C-carbon labeling; $^{11}$C-, $^{13}$C- or $^{14}$C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic or receptor study. The various deuterated forms of the compounds of the present disclosure mean that each available hydrogen atom connected to a carbon atom may be independently replaced with a deuterium atom. Those skilled in the art are able to synthesize the compounds in deuterated form with reference to the relevant literature. Commercially available deuterated starting materials can be used in preparing the deuterated compounds, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain comparable activity to non-deuterated compounds and can achieve better metabolic stability when deuterated at certain specific sites, thereby achieving certain therapeutic advantages.

"Optional" or "optionally" means that the event or circumstance subsequently described may, but not necessarily, occur, and that the description includes instances where the event or circumstance occurs or does not occur. For example, "a heterocyclyl group optionally substituted with alkyl" means that the alkyl may be, but not necessarily, present, and includes instances where the heterocyclyl group is or is not substituted with the alkyl.

"Substituted" means that one or more, preferably 1-5, more preferably 1-3 hydrogen atoms in the group are independently substituted with a corresponding number of substituents. Those skilled in the art are able to determine (experimentally or theoretically) possible or impossible substitution without undue effort. For example, it may be unstable when amino or hydroxy having a free hydrogen is bound to a carbon atom having an unsaturated (e.g., olefinic) bond.

"Pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activities.

The term "pharmaceutically acceptable" used herein means that those compounds, materials, compositions and/or dosage forms that are, within the scope of reasonable medical judgment, suitable for use in contact with the tissues of subjects without excessive toxicity, irritation, allergic reaction, or other problems or complications, and are commensurate with a reasonable benefit/risk ratio and effective for the intended use.

As used herein, the singular forms "a", "an" and "the" include plural references and vice versa, unless otherwise clearly defined in the context.

When the term "about" is applied to parameters such as pH, concentration and temperature, it means that the parameter may vary by $\pm$10%, and sometimes more preferably within $\pm$5%. As will be appreciated by those skilled in the art, when the parameters are not critical, the numbers are generally given for illustrative purposes only and are not intended to be limiting.

The term "linker unit", "linking unit" or "linking fragment" refers to a chemical structural fragment or bond that is linked to a ligand (an antibody, in the present disclosure) at one end and linked to a drug at the other end or that is linked to other linkers before being linked to the drug.

The linker may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), and those derived from coupling to a linker reagent: N-succinimidyl 4-(2-pyridylthio)pentanoate ("SPP"), N-succinimidyl 4-(N-maleimidomethyl)cyclohexane-1 carboxylate ("SMCC", also referred to herein as "MCC"), and N-succinimidyl(4-iodoacetyl)aminobenzoate ("SIAB"). The linker may include extenders, spacers and amino acid units, and may be synthesized using methods known in the art, such as those described in US2005-0238649A1. The linker may be a "cleavable linker" favoring the release of drugs in cells. For example, acid-labile linkers (e.g., hydrazones), proteasesensitive (e.g., peptidase-sensitive) linkers, photolabile linkers, dimethyl linkers or disulfide-containing linkers can be used (Chari et al., Cancer Research, 52: 127-131(1992); U.S. Patent No. 5,208,020).

Linker components include, but are not limited to:

MC = 6-maleimidocaproyl, with a structure shown as follows:

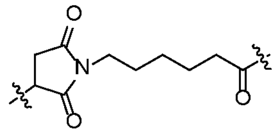

Val-Cit or "vc" = valine-citrulline (an exemplary dipeptide in a protease cleavable linker) citrulline = 2-amino-5-ureidopentanoic acid
PAB = p-aminobenzyloxycarbonyl (an example of "self-immolative" linker components) Me-Val-Cit = N-methyl-valine-citrulline (wherein the linker peptide bond has been modified to prevent it from being cleaved by cathepsin B)
MC(PEG)6-OH = maleimidocaproyl-polyethylene glycol (attachable to antibody cysteine)
SPP = *N*-succinimidyl 4-(2-pyridylthio)valerate
SPDP = *N*-succinimidyl 3-(2-pyridyldithio)propionate
SMCC = succinimidyl-4-(*N*-maleimidomethyl)cyclohexane- 1 -carboxylate
IT = iminothiolane

For the preparation of conventional pharmaceutical compositions, refer to Chinese Pharmacopoeia.

The term "carrier" for the drug of the present disclosure refers to a system that can alter how the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

The term "excipient" is an addition, besides the main drug, to a pharmaceutical formulation. It may also be referred to as an auxiliary material. For example, binders, fillers, disintegrants and lubricants in tablets; the matrix part in semisolid ointment and cream preparations; preservatives, antioxidants, corrigents, fragrances, cosolvents, emulsifiers, solubilizers, osmotic pressure regulators, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus to facilitate the preparation of the tablet. Examples include starch, lactose, inorganic salts of calcium, microcrystalline cellulose and the like.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activities.

The pharmaceutical composition may be in the form of a sterile injectable aqueous solution. Available and acceptable vehicles or solvents include water, Ringer's solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections.

"Administering", "giving" and "treating", when applied to animals, humans, experimental subjects, cells, tissues, organs or biological fluid, refer to contact of an exogenous drug, a therapeutic agent, a diagnostic agent or composition

with the animals, humans, subjects, cells, tissues, organs or biological fluid. "Administering", "giving" and "treating" can refer to, for example, therapeutic, pharmacokinetic, diagnostic, research and experimental methods. The treatment of cells comprises making the reagent in contact with the cells and making the reagent in contact with fluid, where the fluid is in contact with the cells. "Administering", "giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications. The term "pharmaceutically acceptable salt" refers to a salt of the antibody-drug conjugate of the present disclosure, or a salt of the compound of the present disclosure. Such salts are safe and effective when used in mammalian animals and possess the required biological activity. The antibody drug conjugate of the present disclosure contains at least one amino group, and thus can form a salt with an acid. Non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and p-toluenesulfonate.

"Treatment" refers to administering a therapeutic agent, such as a composition comprising any one of the conjugation compounds of the present disclosure, either internally or externally to a subject with one or more symptoms of a disease on which the therapeutic agent is known to have a therapeutic effect. Generally, the therapeutic agent is administered in an amount effective to alleviate one or more symptoms of the disease in the subject or population being treated to induce regression of such symptoms or inhibiting the development of such symptoms to any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating the symptoms of each disease of interest, they shall alleviate the symptoms of the disease of interest in a statistically significant number of subjects as determined by any statistical test method known in the art, such as the Student's t-test, chi-square test, U-test by Mann and Whitney, Kruskal-Wallis test (H-test), Jonckheere-Terpstra test and Wilcoxon test.

One or more embodiments of the present disclosure are described in detail in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

The present invention is further described below with reference to examples, but these examples are not intended to limit the scope of the present invention.

Experimental procedures without conditions specified in the Examples or Test Examples of the present disclosure are generally conducted according to conventional conditions, or according to conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated were commercially available conventional reagents.

## Examples

### Example 1: Construction of Cell Strains Expressing HER3 at High Levels

pCDH-Her3 lentiviral expression vector plasmids, pVSV-G and pCMV-dR8.91 lentiviral system packaging vectors were transfected into viral packaging cells 293T using Lipofectamine 3000 transfection reagent. The medium supernatant containing viruses was collected, filtered, and centrifuged at ultra-high speed. Chinese hamster ovary cells CHO-K1 were allowed to be infected with the concentrated virus, screened using puromycin for two to three weeks, and subjected to FACS single-cell sorting.

According to the HER3 expression levels on the surface of CHO-K1 cells infected with lentivirus determined by FACS, monoclonal cell strains expressing HER3 at high levels were selected.

The selected monoclonal cell strains were expanded and stored for subsequent experiments.

Amino acid sequence of HuMan ErbB3 (UniProtKB - P21860-1, AA Ser 20 - Thr 643)

SEVGNSQAVCPGTLNGLSVTGDAENQYQTLYKLYERCEVVMGNLEIVLTGHNA
DLSFLQWIREVTGYVLVAMNEFSTLPLPNLRVVRGTQVYDGKFAIFVMLNYNT
NSSHALRQLRLTQLTEILSGGVYIEKNDKLCHMDTIDWRDIVRDRDAEIVVKDN
GRSCPPCHEVCKGRCWGPGSEDCQTLTKTICAPQCNGHCFGPNPNQCCHDECA
GGCSGPQDTDCFACRHFNDSGACVPRCPQPLVYNKLTFQLEPNPHTKYQYGGV
CVASCPHNFVVDQTSCVRACPPDKMEVDKNGLKMCEPCGGLCPKACEGTGSG
SRFQTVDSSNIDGFVNCTKILGNLDFLITGLNGDPWHKIPALDPEKLNVFRTVREI
TGYLNIQSWPPHMHNFSVFSNLTTIGGRSLYNRGFSLLIMKNLNVTSLGFRSLKE
ISAGRIYISANRQLCYHHSLNWTKVLRGPTEERLDIKHNRPRRDCVAEGKVCDP
LCSSGGCWGPGPGQCLSCRNYSRGGVCVTHCNFLNGEPREFAHEAECFSCHPE
CQPMEGTATCNGSGSDTCAQCAHFRDGPHCVSSCPHGVLGAKGPIYKYPDVQN
ECRPCHENCTQGCKGPELQDCLGQTLVLIGKTHLT

SEQ ID NO: 1

Nucleotide sequence of HuMan ErbB3

AGCGAAGTCGGCAACAGCCAAGCCGTCTGTCCCGGCACACTCAATGGACTG
TCCGTGACTGGCGACGCCGAGAACCAATACCAGACACTCTACAAGCTCTACG
AGAGGTGCGAGGTGGTCATGGGAAATCTGGAGATCGTGCTGACTGGCCATAA
CGCCGATCTGTCCTTTCTGCAGTGGATTAGGGAAGTGACTGGCTACGTGCTG
GTCGCCATGAATGAGTTTTCCACTCTGCCACTGCCAAATCTGAGAGTGGTGA
GGGGCACTCAAGTGTACGACGGCAAGTTCGCCATTTTCGTCATGCTCAACTA
CAACACAAACTCCAGCCACGCCCTCAGACAGCTGAGGCTCACTCAGCTGAC
AGAAATTCTGTCCGGCGGCGTCTATATCGAGAAAAACGATAAACTGTGCCAC
ATGGACACAATCGATTGGAGGGACATCGTGAGGGATAGGGATGCCGAGATCG
TGGTCAAGGATAACGGAAGGAGCTGTCCTCCTTGTCATGAGGTCTGCAAGGG
AAGGTGTTGGGGACCCGGCTCCGAAGACTGCCAGACACTGACTAAGACTAT
CTGCGCCCCTCAGTGCAATGGACACTGCTTCGGCCCAAATCCAAACCAGTGC
TGCCACGACGAATGTGCCGGCGGATGCAGCGGACCACAAGATACAGACTGC
TTCGCTTGTAGACACTTCAATGACTCCGGCGCTTGTGTGCCTAGGTGTCCACA
GCCACTCGTGTACAACAAGCTCACTTTTCAGCTCGAGCCTAACCCTCACACT
AAGTACCAATACGGCGGAGTCTGCGTCGCCAGCTGTCCTCACAACTTCGTGG
TGGATCAGACAAGCTGCGTGAGAGCTTGCCCTCCAGATAAAATGGAGGTGG
ACAAGAACGGACTGAAGATGTGTGAGCCTTGCGGCGGACTGTGTCCTAAAG
CTTGCGAGGGCACTGGCTCCGGATCTAGGTTCCAGACTGTCGACTCCAGCAA
CATCGACGGCTTTGTGAACTGCACTAAGATTCTGGGCAATCTGGACTTTCTGA
TCACTGGCCTCAACGGCGATCCTTGGCACAAGATCCCAGCTCTGGACCCAGA
AAAGCTGAATGTGTTTAGGACAGTGAGGGAGATTACTGGCTACCTCAACATC

CAGAGCTGGCCTCCACACATGCACAACTTCAGCGTGTTCTCCAATCTGACTA
CAATCGGCGGCAGATCCCTCTATAATAGGGGCTTCTCTCTGCTCATCATGAAG
AATCTGAACGTCACTTCTCTGGGCTTCAGATCTCTGAAGGAGATCTCCGCCG
GAAGGATTTACATCTCCGCCAATAGGCAGCTCTGTTACCACCACAGCCTCAA
CTGGACTAAGGTGCTGAGGGGACCTACTGAGGAAAGGCTGGACATTAAACA
CAATAGGCCAAGAAGGGATTGCGTCGCTGAGGGCAAAGTGTGTGATCCTCTG
TGTAGCTCCGGAGGATGTTGGGGACCCGGCCCCGGCCAGTGTCTGAGCTGTA
GGAATTATTCTAGGGGCGGCGTGTGTGTGACACACTGCAACTTTCTGAACGG
CGAACCTAGGGAATTCGCCCATGAAGCCGAGTGCTTCAGCTGCCACCCAGAG
TGTCAGCCTATGGAGGGCACAGCTACATGCAATGGCAGCGGATCCGACACAT
GTGCTCAGTGTGCCCACTTTAGGGATGGACCTCATTGCGTCAGCAGCTGTCC
ACACGGCGTGCTGGGAGCCAAGGGCCCTATCTACAAGTACCCAGATGTGCAG
AACGAGTGTAGGCCTTGCCACGAGAATTGCACACAAGGCTGCAAGGGCCCA
GAGCTGCAAGATTGCCTCGGCCAGACTCTGGTGCTCATCGGCAAGACTCATC
TCACT

SEQ ID NO: 2

**Example 2: Production of Anti-Human HER3 Monoclonal Antibody**

2.1. Preparation of positive control antibody

A positive control antibody U3 was prepared with reference to WO2007077028A2 (page 118, U1-59). The heavy and light chain amino acid sequences of U3 are as follows:

Heavy chain of U3:

QVQLQQWGAGLLKPSETLSLTCAVYGGSFSGYYWSWIRQPPGKGLEWIGEINH
SGSTNYNPSLKSRVTISVETSKNQFSLKLSSVTAADTAVYYCARDKWTWYFDL
WGRGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK

SEQ ID NO: 3

Light chain of U3 :

DIEMTQSPDSLAVSLGERATINCRSSQSVLYSSSNRNYLAWYQQNPGQPPKLLIY
WASTRESGVPDRFSGSGSGTDFTLTISSLQAEDVAVYYCQQYYSTPRTFGQGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 4

2.2. Preparation of the antibody of the present disclosure

A positive clone was obtained by panning using a fully human natural phage antibody library and antigen Biotinylated HuMan ErbB3 (purchased from Beijing ACROBiosystems Biotech Ltd., catalog No. ER3-H82E6) followed by phage detection by ELISA. The positive clone was sequenced. After the sequence was obtained, the positive clone was inserted into the protein expression vector Phr-IgG and expressed by HEK293 and Expi-CHO-S cells. After purification, FACS and endocytic activity validation assays were performed, and a fully human antibody molecule HER3-29 was obtained.

The antibody constant regions of the fully human antibody molecule HER3-29:

Human IgG1 Heavy chain constant region:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 5

Human κ light chain constant region:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 6

The heavy chain variable region and light chain variable region sequences of the fully human antibody molecule HER3-29 are as follows:

Heavy chain variable region of HER3-29:

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSS

SEQ ID NO: 7

Light chain variable region of HER3-29:

DIQMTQSPSSLSASIGDRATITCRASQHVGTYLNWYQQKPGKTPKLLISGAANL QSGVPSRFSGSGSGTDFTLTISSLQPEDFATYYCQQSYNTPPFSFGQGTKVEIK

SEQ ID NO: 8

The CDR sequences obtained by different numbering schemes are as follows:

Table 1. CDR sequences obtained by the Chothia numbering scheme

| Antibody | HER3-29 | Sequence No. |
|---|---|---|
| **Heavy chain CDR1** | GFTFDDY | SEQ ID NO: 9 |
| **Heavy chain CDR2** | SWNSGS | SEQ ID NO: 10 |
| **Heavy chain CDR3** | EGLPGLDY | SEQ ID NO: 11 |
| **Light chain CDR1** | RASQHVGTYLN | SEQ ID NO: 12 |
| **Light chain CDR2** | GAANLQS | SEQ ID NO: 13 |
| **Light chain CDR3** | QQSYNTPPFS | SEQ ID NO: 14 |

Table 2. CDR sequences obtained by the IMGT numbering scheme

| Antibody | HER3-29 | Sequence No. |
|---|---|---|
| **Heavy chain CDR1** | GFTFDDYA | SEQ ID NO: 15 |
| **Heavy chain CDR2** | ISWNSGSI | SEQ ID NO: 16 |
| **Heavy chain CDR3** | AKEGLPGLD Y | SEQ ID NO: 17 |
| **Light chain CDR1** | QHVGTY | SEQ ID NO: 18 |
| **Light chain CDR2** | GAA | SEQ ID NO: 19 |
| **Light chain CDR3** | QQSYNTPPFS | SEQ ID NO: 20 |

Table 3. CDR sequences obtained by the Kabat numbering scheme

| Antibody | HER3-29 | Sequence No. |
|---|---|---|
| **Heavy chain CDR1** | DYAMH | SEQ ID NO: 21 |
| **Heavy chain CDR2** | GISWNSGSIGYADSVKG | SEQ ID NO: 22 |
| **Heavy chain CDR3** | EGLPGLDY | SEQ ID NO: 23 |
| **Light chain CDR1** | RASQHVGTYLN | SEQ ID NO: 24 |
| **Light chain CDR2** | GAANLQS | SEQ ID NO: 25 |
| **Light chain CDR3** | QQSYNTPPFS | SEQ ID NO: 26 |

The heavy chain and light chain sequences of the fully human antibody molecule HER3-29 are as follows:

Heavy chain of HER3-29:

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK

SEQ ID NO: 27

Light chain of HER3-29:

DIQMTQSPSSLSASIGDRATITCRASQHVGTYLNWYQQKPGKTPKLLISGAANL
QSGVPSRFSGSGSGTDFTLTISSLQPEDFATYYCQQSYNTPPFSFGQGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 28

**Example 3**: **Preparation of ADCs**

Determination of the DAR values of ADCs

The DAR values of the ADCs of the present disclosure were calculated by RP-HPLC (reversed-phase high performance liquid chromatography), specifically as follows:

**1. Determination method:**

A naked antibody and an ADC test sample (at concentration 1 mg/mL) were reduced with 4 μL of DDT (sigma) in a water bath at 37 °C for 1 h, and then transferred to an insert. Analysis was performed on a high performance liquid chromatograph Agilent 1200, with Agilent PLRP-S 1000A 8 μm 4.6 × 250 mm selected as the chromatography column, the column temperature at 80 °C, the DAD detector at wavelength 280 nm, the flowrate at 1 mL/min, and the injection volume at 40 μL. Comparisons were made to the spectra of the sample and the naked antibody to identify the locations of the light chain and heavy chain, and then integration was performed on the spectrum of the test sample to calculate the DAR value.

**2. Preparation of solutions**

1) 0.25 M DTT solution:
Example of preparation: 5.78 mg of DTT was weighed into 150 μL of purified water and completely dissolved to give 0.25 M DTT solution, which was then stored at -20 °C.
2) Mobile phase A (0.1% TFA in water):
Example of preparation: 1000 mL of purified water was measured out using a graduated cylinder, and 1 mL of TFA (sigma) was added. The solution was well mixed before use and was stored at 2-8 °C for 14 days.
3) Mobile phase B (0.1% TFA in acetonitrile):

Example of preparation: 1000 mL of acetonitrile was measured out using a graduated cylinder, and 1 mL of TFA was added. The solution was well mixed before use and was stored at 2-8 °C for 14 days.

**3. Data analysis**

Comparisons were made to the spectra of the sample and the naked antibody to identify the locations of the light chain and heavy chain, and then integration was performed on the spectrum of the test sample to calculate the DAR value.

The calculation formula is as follows:

| Name | Number of linked drugs |
|---|---|
| LC | 0 |
| LC+1 | 2 |
| HC | 0 |
| HC+1 | 2 |
| HC+2 | 4 |
| HC+3 | 6 |

$$\text{Total LC peak area} = \text{LC peak area} + \text{LC+1 peak area}$$

$$\text{Total HC peak area} = \text{HC peak area} + \text{HC+1 peak area} + \text{HC+2 peak area} + \text{HC+3 peak area}$$

$$\text{LC DAR} = \Sigma(\text{number of linked drugs} \times \text{percent peak area})/\text{total LC peak area}$$

$$\text{HC DAR} = \Sigma(\text{number of linked drugs} \times \text{percent peak area})/\text{total HC peak area}$$

$$\text{DAR} = \text{LC DAR} + \text{HC DAR}$$

Drug

The drug moiety of the conjugates of the present disclosure may be any suitable drug. Particularly suitable drugs are described, for example, in PCT Publication No. WO2020063676A1, which is incorporated herein by reference in its entirety. The compound 9A of the present disclosure (i.e., compound 9-A of Example 9 in WO2020063676 A1) is N-((2R,10S)-10-benzyl-2-cyclopropyl-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-1,6,9,12,15-pentaoxo-3-oxa-5,8,11,14-tetraazahexadec-16-yl)-6-(2,5-dioxo-2,5-dihydro-1H-pyrrol-1-yl)hexanamide, which has the structure below:

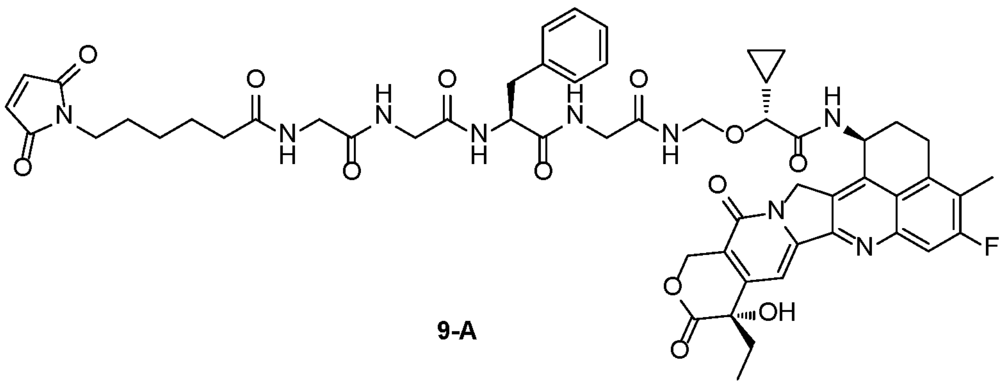

9-A

The present disclosure uses the following methods to prepare antibody-drug conjugates represented by the ADC general formula (HER3-29-9A) by adjusting the reaction parameters.

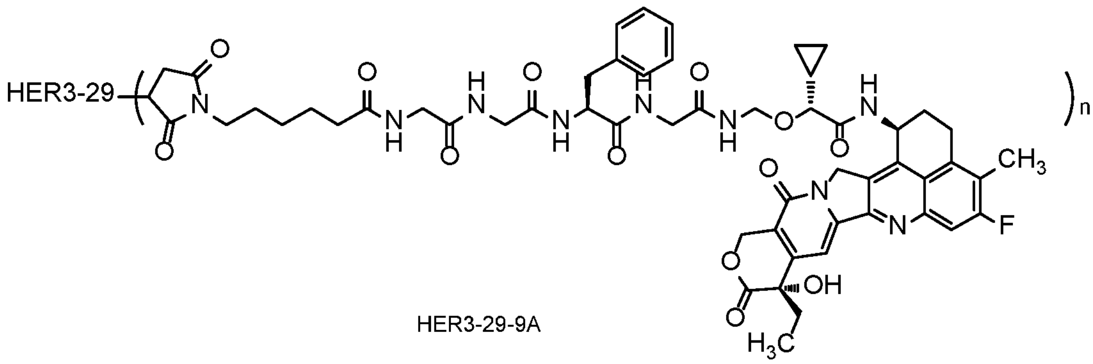

HER3-29-9A

**Example 3-1: ADC-1**

To an aqueous PBS buffer of antibody HER3-29 (0.05 M pH 6.5 aqueous PBS buffer; 10.0 mg/mL, 11.8 mL, 797 nmol) was added at 37 °C a prepared aqueous solution of tris(2-carboxyethyl)phosphine (TCEP) (10 mM, 208.2 μL, 2.082 μMol). The mixture was reacted on a water bath shaker at 37 °C for 3 h, and then the reaction was terminated. The reaction mixture was cooled to 25 °C in a water bath.

Compound **9A** (prepared according to the method for compound 9-A of Example 9 in WO2020063676, which is incorporated in the present disclosure in its entirety) (8.6 mg, 8.006 μmol) was dissolved in 500 μL of DMSO, and the resulting solution was added to the above reaction mixture. The mixture was reacted on a water bath shaker at 25 °C for 3 h, and then the reaction was terminated. The reaction mixture was desalted and purified through a Sephadex G25 gel column (elution phase: 0.05 M pH 6.5 aqueous PBS buffer, containing 0.001 M EDTA) to give an exemplary product of the conjugate HER3-29-9A, **ADC-1,** in a PBS buffer (4.02 mg/mL, 27.9 mL), which was then stored at 4 °C. Mean calculated by RP-HPLC: DAR = 4.19.

**Example 3-2: ADC-2**

To an aqueous PBS buffer of antibody HER3-29 (0.05 M pH 6.5 aqueous PBS buffer; 10.0 mg/mL, 11.8 mL, 797 nmol) was added at 37 °C a prepared aqueous solution of tris(2-carboxyethyl)phosphine (TCEP) (10 mM, 128 μL, 1.281 μmol). The mixture was reacted on a water bath shaker at 37 °C for 3 h, and then the reaction was terminated. The reaction mixture was cooled to 25 °C in a water bath.

Compound **9A** (6.88 mg, 6.405 μmol) was dissolved in 400 μL of DMSO, and the resulting solution was added to the above reaction mixture. The mixture was reacted on a water bath shaker at 25 °C for 3 h, and then the reaction was terminated. The reaction mixture was desalted and purified through a Sephadex G25 gel column (elution phase: 0.05 M pH 6.5 aqueous PBS buffer, containing 0.001 M EDTA) to give an exemplary product of the conjugate HER3-29-9A, **ADC-2,** in a PBS buffer (4.24 mg/mL, 27.2 mL), which was then stored at 4 °C. Mean calculated by RP-HPLC: DAR = 2.91.

**Example 3-3: ADC-3**

To an aqueous PBS buffer of antibody HER3-29 (0.05 M pH 6.5 aqueous PBS buffer; 10.0 mg/mL, 3.1 mL, 209 nmol) was added at 37 °C a prepared aqueous solution of tris(2-carboxyethyl)phosphine (TCEP) (10 mM, 111 μL, 1.111 μMol). The mixture was reacted on a water bath shaker at 37 °C for 3 h, and then the reaction was terminated. The reaction mixture was cooled to 25 °C in a water bath.

Compound **9A** (3.37 mg, 3.137 μmol) was dissolved in 120 μL of DMSO, and the resulting solution was added to the above reaction mixture. The mixture was reacted on a water bath shaker at 25 °C for 3 h, and then the reaction was terminated. The reaction mixture was desalted and purified through a Sephadex G25 gel column (elution phase: 0.05 M pH 6.5 aqueous PBS buffer, containing 0.001 M EDTA) to give an exemplary product of the conjugate HER3-29-9A, **ADC-3,** in a PBS buffer (1.48 mg/mL, 12.8 mL), which was then stored at 4 °C. Mean calculated by RP-HPLC: DAR = 7.27.

**Example 3-4: ADC-4**

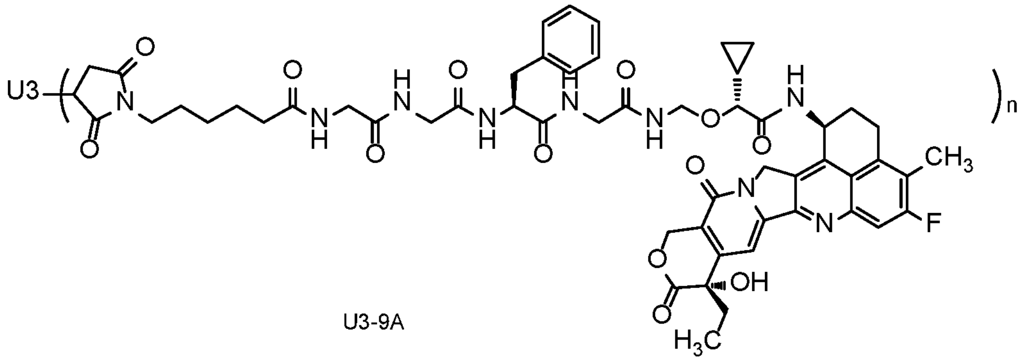

U3-9A

To an aqueous PBS buffer of antibody U3 (0.05 M pH 6.5 aqueous PBS buffer; 10.0 mg/mL, 3.1 mL, 209 nmol) was added at 37 °C a prepared aqueous solution of tris(2-carboxyethyl)phosphine (TCEP) (10 mM, 111 μL, 1.111 μmol). The mixture was reacted on a water bath shaker at 37 °C for 3 h, and then the reaction was terminated. The reaction mixture was cooled to 25 °C in a water bath.

Compound **9A** (3.37 mg, 3.137 μmol) was dissolved in 120 μL of DMSO, and the resulting solution was added to the above reaction mixture. The mixture was reacted on a water bath shaker at 25 °C for 3 h, and then the reaction was terminated. The reaction mixture was desalted and purified through a Sephadex G25 gel column (elution phase: 0.05 M pH 6.5 aqueous PBS buffer, containing 0.001 M EDTA) to give an exemplary product of the conjugate U3-9A, **ADC-4,** in a PBS buffer (1.48 mg/mL, 12.8 mL), which was then stored at 4 °C. Mean calculated by RP-HPLC: DAR = 6.76.

**Example 3-5: ADC-5**

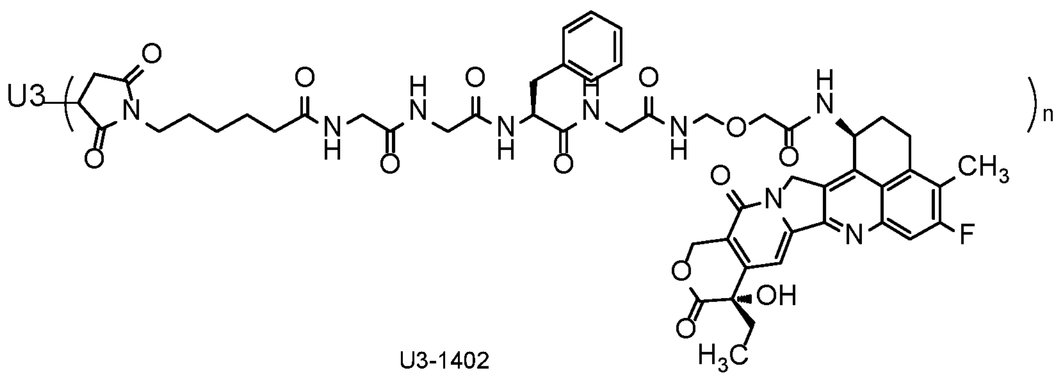

U3-1402

With reference to Example 12 on page 156 of the specification of WO2015155998A1, U3-1402 was prepared as a positive control. To an aqueous PBS buffer of antibody U3 (0.05 M pH 6.5 aqueous PBS buffer; 10.0 mg/mL, 3.1

mL, 236 nmol) was added at 37 °C a prepared aqueous solution of tris(2-carboxyethyl)phosphine (TCEP) (10 mM, 130 μL). The mixture was reacted on a water bath shaker at 37 °C for 3 h, and then the reaction was terminated. The reaction mixture was cooled to 25 °C in a water bath.

Compound **1402** (3.67 mg, 3.54 μmol) was dissolved in 180 μL of DMSO, and the resulting solution was added to the above reaction mixture. The mixture was reacted on a water bath shaker at 25 °C for 3 h, and then the reaction was terminated. The reaction mixture was desalted and purified through a Sephadex G25 gel column (elution phase: 0.05 *M* pH 6.5 aqueous PBS buffer, containing 0.001 M EDTA) to give an exemplary product of the conjugate U3-1402, **ADC-5,** in a PBS buffer (1.53 mg/mL, 15.4 mL), which was then stored at 4 °C. Mean calculated by RP-HPLC: DAR = 6.97.

## Test Examples

### Test Example 1: Binding of Antibodies to Free HER3 Protein

HER3 protein was diluted to 1 μg/mL with pH 7.4 PBS buffer (Shanghai BasalMedia Technologies Co., LTD., B320) and added to a 96-well microplate at 100 μL/well. The plate was incubated at 4 °C overnight. After the liquid was discarded, 300 μL of 5% skim milk (BD, 232100) diluted with PBS was added to each well for blocking, and the plate was incubated at 37 °C for 2 h. After the blocking was completed, the blocking solution was discarded; after the plate was washed 3 times with PBST buffer (pH 7.4 PBS containing 0.1% tween-20), 100 μL of a gradient diluted antibody solution was added to each well, and the plate was incubated at 37 °C for 1 h. After the incubation was completed, the plate was washed 3 times with PBST buffer, and 100 μL of 1:8000 diluted Mouse Anti-HuMan IgG (H+L) (Jackson ImmunoResearch, 209-035-088) was added to each well. The plate was incubated at 37 °C for 1 h. After the plate was washed 3 times with PBST buffer, 100 μL of TMB chromogenic substrate (KPL, 5120-0077) was added to each well, and the plate was incubated at room temperature for 10-15 min. The reaction was terminated by adding 50 μL of 1 M $H_2SO_4$ to each well. The absorbance readings at 450 nm were taken on a microplate reader, and the binding curves of the antibodies to antigen were fitted with software, as shown in FIG. 1. The $EC_{50}$ values were calculated and the results are shown in Table 4.

Table 4. Binding activity of antibodies to HER3 protein

| Antibody | HER3-29 | U3 |
|---|---|---|
| $EC_{50}$ (nM) | 0.14 | 0.56 |
| Conclusion: the antibody HER3-29 of the present disclosure has better binding activity to HER3 protein than the control antibody U3. | | |

### Test Example 2: Binding of Antibodies to Cells Expressing HER3

MCF7 cells (ATCC, HTB-22) were suspended in FACS buffer (2% fetal bovine serum (Gibco, 10099141) pH 7.4 PBS (Sigma, P4417-100TAB)) to give a $1\times10^6$ cells/mL cell suspension, which was then added to a 96-well round-bottom plate at 100 μL/well. After centrifugation and removal of the supernatant, the test antibody that was diluted with FACS buffer to different concentrations was added at 50 μL/well. The plate was incubated in the dark in a 4 °C refrigerator for 1 h. The plate was washed 3 times with FACS buffer by centrifugation at 300 g, and Alexa Fluor 488 Goat anti-HuMan IgG (H+L) (invitrogen, A-11013) at working concentration was then added. The plate was incubated in the dark in a 4 °C refrigerator for 40 min. The plate was washed 3 times with FACS buffer by centrifugation at 300 g and tested on a BD FACSCantoII flow cytometer for geometric mean fluorescence intensity. The results are shown in FIG. 2. The $EC_{50}$ values are shown in Table 5.

Table 5. Antibody cell-level binding activity

| Antibody | HER3-29 | U3 |
|---|---|---|
| $EC_{50}$ (nM) | 0.057 | 0.249 |
| Conclusion: the antibody HER3-29 of the present disclosure has better binding activity to cells expressing HER3 protein than the control antibody U3. | | |

### Test Example 3: DT3C Antibody Endocytosis Assay

The purpose of this assay is that the activated diphtheria toxin (DT) kills cells after the DT3C protein enters the

cells, indirectly reflecting endocytosis of the HER3 antibody. The *in vitro* endocytic activity of the antibody was evaluated according to $IC_{50}$ and Imax. DT3C is a recombinantly expressed fusion protein formed by fusion of Fragment A (toxin-only portion) of diphtheria toxin to fragment 3C (IgG binding portion) of group G streptococcus. The protein has high affinity for the Fc structure of an antibody and enters cells with the antibody when the antibody is endocytosed. Under the action of intracellular furin, toxic DT is released. DT can inhibit the EF2-ADP ribosylation activity, block the protein translation process and finally cause cell death. DT3C that does not enter the cell has no activity of cell killing. The endocytic activity of the antibody was evaluated according to cell killing.

A $2\times10^4$ cells/mL suspension of CHOK1 cells recombinantly expressing HER3 was prepared with fresh cell medium containing 20% low IgG FBS and added to a cell culture plate at 50 μL/well. The plate was incubated at 37 °C with 5% carbon dioxide for 16 h. DT3C at 4× concentration was formulated in serum-free medium and filtered through a 0.22 μm filter to obtain a sterile solution. Antibody at 4× concentration was prepared in serum-free medium, and 80 μL of DT3C (400 nM) and 80 μL of antibody (66 nM) were mixed at a volume of 1: 1, and incubated at room temperature for 30 min. To 50 μL of cells, 50 μL of the diluted antibody was added. The cells were incubated in an incubator for three days. To each well was added 50 μL CTG (CellTiter-Glo™ reagent, G7573). The plate was incubated at room temperature in the dark for 10 min. The chemiluminescence readings were taken on Victor3. The results are shown in FIG. 3 and Table 6.

Table 6. Endocytic activity of antibodies

| Antibody | HER3-29 | U3 |
|---|---|---|
| Imax | 48% | 19% |
| $IC_{50}$ (nM) | 0.51 | 2.81 |
| Conclusion: the antibody HER3-29 of the present disclosure has better cellular endocytic activity than the control antibody U3. | | |

**Test Example 4: pHrodo Antibody Endocytosis Assay**

The purpose of this assay is to reflect the endocytosis of the HER3 antibody according to changes in the fluorescence signal following internalization of the dye. The *in vitro* endocytic activity of the antibody was evaluated according to the intensity of the fluorescent signal.

Fab fragments coupled with the pH sensitive pHrodo iFL dye can bind directly to the Fc region of the HER3 antibody without affecting the antigen recognition of the antibody. The pHrodo iFL dye hardly fluoresces at neutral pH. When the HER3 antibody is endocytosed, the dye is internalized at the same time. The fluorescence signal will gradually intensify as the pH decreases. The endocytic activity of the antibody was evaluated according to how the fluorescence signal intensified.

HER3/CHOK1 cells were cultured with DMEM/F12+10% FBS+10 μg/mL puromycin. On the first day of the experiment, a $2\times10^5$ cells/mL cell suspension was prepared with fresh cell-containing medium and added to a 96-well cell culture plate at 100 μL/well. The plate was cultured at 37 °C with 5% carbon dioxide for 24 h.

50 μL of the cell broth was sucked out of the plate, and 50 μL of a mixture of antibody and pHrodo dye was added to each well. Two replicate wells were set for each antibody sample. A dye addition-only group and an isotype IgG1 control group were set.

After 24 hours of culture in an incubator, the medium was sucked out, and the cells in each well were digested with 50 μL of pancreatin for 2 min. The digestion was stopped with 50 μL of fresh medium. The cells from the replicate wells of the same sample were transferred to a well of a round-bottom plate using a multi-channel pipette. The cells were centrifuged at 1500 rpm for 2 min, and the medium was discarded. The cells were washed once with FACS Buffer (PBS+2.5%FBS) and centrifuged at 1500 rpm for 2 min. The cells were resuspended by adding 200 μL of FACS Buffer (PBS+2.5% FBS), and the FITC signal was detected by flow cytometry. The data were analyzed using Flowjo 7.6. The results are shown in FIG. 4 and Table 7.

Table 7. Endocytic activity of antibodies

| Antibody | HER3-29 | U3 |
|---|---|---|
| FITC signal | 373 | 267 |
| Conclusion: the antibody HER3-29 of the present disclosure has better cellular endocytic activity than the control antibody U3. | | |

**Test Example 5: Cell Activity Assay of ADC Molecules**

The purpose of this assay is to determine the killing effects of the ADC samples on cells and to evaluate the *in vitro* activity of Her3-ADC according to $IC_{50}$ and Imax.

MCF7 cells (human breast cancer cells), SW620 cells (human colon cancer cells, Nanjing Cobioer, CBP60036) and WiDr cells (human colon cancer cells) were digested with pancreatin, neutralized with fresh medium, centrifuged at 1000 rpm, then resuspended in medium, and counted. Then the cell suspensions were adjusted to a density of 500 cells/well and added to a 96-well cell culture plate. No cell but only 135 μL of medium was added to the wells in the 11th column. The cells were cultured at 37 °C with 5% carbon dioxide for 16 h.

An ADC sample was diluted with PBS to 15 μM (10× concentration). This concentration was taken as the initial concentration, and the sample was 5-fold diluted with PBS to a total of 8 concentrations. To each well, 15 μL of the solution with 10× concentration was added. The cells were cultured at 37 °C with 5% carbon dioxide for 6 days.

To each well, 70 μL of CTG was added. The plate was incubated at room temperature in the dark for 10 min. A white membrane was attached to the bottom of the cell culture plate, and the chemiluminescence readings were taken on Victor3. The data from this assay was processed using the data processing software GraphPad prism5.0. The results are shown in Table 8.

Table 8. *In vitro* killing assay of HER3-29-9A with different DAR values

| ADC sample | DAR value | Test cell | | | | | |
|---|---|---|---|---|---|---|---|
| | | MCF7 | | SW620 | | WiDr | |
| | | $IC_{50}$ (nM) | Imax % | $IC_{50}$ (nM) | Imax % | $IC_{50}$ (nM) | Imax % |
| ADC-5 | 6.97 | 95.52 | 85.53 | 110.5 | 99.04 | 420.6 | 97.98 |
| ADC-4 | 6.76 | 68.2 | 83.66 | 90.2 | 100.06 | 327.5 | 100.89 |
| ADC-3 | 7.27 | 22.35 | 86.72 | 47.27 | 100.06 | 143.5 | 100.54 |
| ADC-1 | 4.19 | 71.51 | 91.41 | 90.47 | 100.35 | 673.4 | 94.1 |
| ADC-2 | 2.91 | 119.6 | 88.91 | 130.4 | 99.85 | 2278 | 87.9 |
| Conclusion: the ADC samples ADC-1, ADC-2 and ADC-3 of the present disclosure has better killing activity on cells than the positive controls ADC-4 and ADC-5. | | | | | | | |

Biological Evaluation ***of In Vivo* Activity**

**Test Example 6: In Vivo Efficacy Evaluation of HER3 high-expression CDX model**

SW620 cells ($5\times10^6$ cells/mouse) were inoculated subcutaneously into the right flank of Balb/c nude mice, and after 7 days, the mice were divided into a total of 9 groups of 8. The mean grouping volume was 134.75 $mm^3$. ADC was intraperitoneally injected once every 5 days and was administered 3 times in total. The injections were administered at a dose of 0.1 mL/10 g body weight/mouse. The tumor volumes and body weights were measured twice a week and the results were recorded. Data were recorded using Excel statistical software: the mean values were calculated as avg; the SD values were calculated as STDEV; the SEM values were calculated as STDEV/SQRT (number of animals per group); GraphPad Prism software was used for plot, and statistical analysis of the data was performed using Two-way ANOVA or One-way ANOVA.

Tumor volume (V) was calculated as: $V = 1/2 \times L_{long} \times L_{short}^2$

The relative tumor proliferation rate T/C (%) = $(T - T_0)/(C - C_0) \times 100\%$, where T and C are the tumor volume of animals at the end of the experiment in the treatment group and control group, respectively; $T_0$ and $C_0$ are the tumor volume of animals at the beginning of the experiment in the treatment group and control group, respectively.

Tumor inhibition rate TGI (%) = 1 - T/C (%). The results are shown in FIG. 5 and Table 9.

Table 9. Efficacy of ADCs on SW620 xenograft tumors in tumor-bearing nude mice

| ADC sample | DAR value | Tumor inhibition rate TGI (%) | | |
|---|---|---|---|---|
| | | 6mpk | 3mpk | 1.5mpk |
| ADC-5 | 6.97 | 98.8 | 56.6 | 24.1 |

(continued)

| ADC sample | DAR value | Tumor inhibition rate TGI (%) | | |
|---|---|---|---|---|
| | | 6mpk | 3mpk | 1.5mpk |
| ADC-1 | 4.19 | - | 72.5 | 22.4 |
| ADC-2 | 2.91 | 86.3 | 65.6 | 6.3 |
| Conclusion: the ADC-1 and ADC-2 of the present disclosure has better efficacy on SW620 xenograft tumors in tumor-bearing mice than the positive control ADC-5. | | | | |

## Claims

1. An isolated anti-HER3 antibody, wherein the anti-HER3 antibody has one or more of the following characteristics:

a. the anti-HER3 antibody binds to HER3 protein with an apparent affinity $EC_{50}$ of less than 0.5 nM, as determined by ELISA;
b. the anti-HER3 antibody binds to HER3 protein expressed by MCF7 cells with an apparent affinity $EC_{50}$ of less than 0.2 nM, as determined by FACS;
c. the anti-HER3 antibody can be endocytosed by cells expressing human HER3; preferably, the anti-HER3 antibody has an $IC_{50}$ of less than 2 nM, as determined through a DT3C antibody endocytosis assay;
d. the anti-HER3 antibody can be endocytosed by cells expressing human HER3; preferably, the anti-HER3 antibody has an FITC signal of greater than 300, as determined through a pHrodo antibody endocytosis assay.

2. The isolated anti-HER3 antibody according to claim 1, wherein the anti-HER3 antibody comprises: 1) HCDR1, HCDR2 and HCDR3 comprised in a heavy chain variable region set forth in SEQ ID NO: 7; and 2) LCDR1, LCDR2 and LCDR3 comprised in a light chain variable region set forth in SEQ ID NO: 8;
preferably, the anti-HER3 antibody comprises a heavy chain variable region and a light chain variable region, wherein:

a. the heavy chain variable region comprises HCDR1, HCDR2 and HCDR3 set forth in SEQ ID NO: 9, SEQ ID NO: 10 and SEQ ID NO: 11, respectively, and the light chain variable region comprises LCDR1, LCDR2 and LCDR3 set forth in SEQ ID NO: 12, SEQ ID NO: 13 and SEQ ID NO: 14, respectively;
wherein the CDR regions are defined according to the Chothia numbering scheme; or
b. the heavy chain variable region comprises HCDR1, HCDR2 and HCDR3 set forth in SEQ ID NO: 15, SEQ ID NO: 16 and SEQ ID NO: 17, respectively, and the light chain variable region comprises LCDR1, LCDR2 and LCDR3 set forth in SEQ ID NO: 18, SEQ ID NO: 19 and SEQ ID NO: 20, respectively;
wherein the CDR regions are defined according to the IMGT numbering scheme; or
c. the heavy chain variable region comprises HCDR1, HCDR2 and HCDR3 set forth in SEQ ID NO: 21, SEQ ID NO: 22 and SEQ ID NO: 23, respectively, and the light chain variable region comprises LCDR1, LCDR2 and LCDR3 set forth in SEQ ID NO: 24, SEQ ID NO: 25 and SEQ ID NO: 26, respectively;
wherein the CDR regions are defined according to the Kabat numbering scheme.

3. An isolated anti-HER3 antibody, wherein the anti-HER3 antibody comprises a heavy chain variable region and a light chain variable region, wherein:

a. the heavy chain variable region comprises HCDR1, HCDR2 and HCDR3 set forth in SEQ ID NO: 9, SEQ ID NO: 10 and SEQ ID NO: 11, respectively, and the light chain variable region comprises LCDR1, LCDR2 and LCDR3 set forth in SEQ ID NO: 12, SEQ ID NO: 13 and SEQ ID NO: 14, respectively;
the CDR regions described above are defined according to the Chothia numbering scheme; or
b. the heavy chain variable region comprises HCDR1, HCDR2 and HCDR3 set forth in SEQ ID NO: 15, SEQ ID NO: 16 and SEQ ID NO: 17, respectively, and the light chain variable region comprises LCDR1, LCDR2 and LCDR3 set forth in SEQ ID NO: 18, SEQ ID NO: 19 and SEQ ID NO: 20, respectively;
the CDR regions described above are defined according to the IMGT numbering scheme; or
c. the heavy chain variable region comprises HCDR1, HCDR2 and HCDR3 set forth in SEQ ID NO: 21, SEQ ID NO: 22 and SEQ ID NO: 23, respectively, and the light chain variable region comprises LCDR1, LCDR2 and LCDR3 set forth in SEQ ID NO: 24, SEQ ID NO: 25 and SEQ ID NO: 26, respectively;
the CDR regions described above are defined according to the Kabat numbering scheme.

**4.** The isolated anti-HER3 antibody according to any one of claims 1 to 3, wherein the anti-HER3 antibody is a human antibody or an antigen-binding fragment.

**5.** The isolated anti-HER3 antibody according to any one of claims 1 to 4, comprising a heavy chain variable region and a light chain variable region, wherein:

the heavy chain variable region has an amino acid sequence having at least 90% sequence identity to SEQ ID NO: 7, and/or the light chain variable region has an amino acid sequence having at least 90% sequence identity to SEQ ID NO: 8;
preferably, the anti-HER3 antibody comprises a heavy chain variable region and a light chain variable region, wherein:
the heavy chain variable region has an amino acid sequence set forth in SEQ ID NO: 7, and the light chain variable region has an amino acid sequence set forth in SEQ ID NO: 8.

**6.** The isolated anti-HER3 antibody according to any one of claims 1 to 5, comprising:

a heavy chain having at least 85% sequence identity to SEQ ID NO: 27, and/or a light chain having at least 85% sequence identity to SEQ ID NO: 28;
preferably, the anti-HER3 antibody comprising:
a heavy chain set forth in SEQ ID NO: 27 and a light chain set forth in SEQ ID NO: 28.

**7.** The isolated anti-HER3 antibody according to any one of claims 1 to 6, wherein the anti-HER3 antibody has one or more of the following characteristics:

a. the anti-HER3 antibody binds to HER3 protein with an apparent affinity $EC_{50}$ of less than 0.5 nM, as determined by ELISA;
b. the anti-HER3 antibody binds to HER3 protein expressed by MCF7 cells with an apparent affinity $EC_{50}$ of less than 0.2 nM, as determined by FACS;
c. the anti-HER3 antibody can be endocytosed by cells expressing human HER3; preferably, the anti-HER3 antibody has an $IC_{50}$ of less than 2 nM, as determined through a DT3C antibody endocytosis assay;
d. the anti-HER3 antibody can be endocytosed by cells expressing human HER3; preferably, the anti-HER3 antibody has an FITC signal of greater than 300, as determined through a pHrodo antibody endocytosis assay.

**8.** An isolated anti-HER3 antibody, wherein the antibody competes for binding to human HER3 with the anti-HER3 antibody according to any one of claims 1 to 7.

**9.** A nucleic acid molecule encoding the isolated anti-HER3 antibody according to any one of claims 1 to 8.

**10.** A host cell comprising the nucleic acid molecule according to claim 9.

**11.** An immunoconjugate comprising the isolated anti-HER3 antibody according to any one of claims 1 to 8 and an effector molecule, wherein the effector molecule is coupled to the anti-HER3 antibody;
preferably, the effector molecule is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

**12.** A method for immunodetection or determination of HER3, comprising a step of contacting the isolated anti-HER3 antibody according to any one of claims 1 to 8 with a subject or a sample from the subject.

**13.** An antibody-drug conjugate of general formula (Pc-$L_a$-Y-D) or a pharmaceutically acceptable salt thereof:

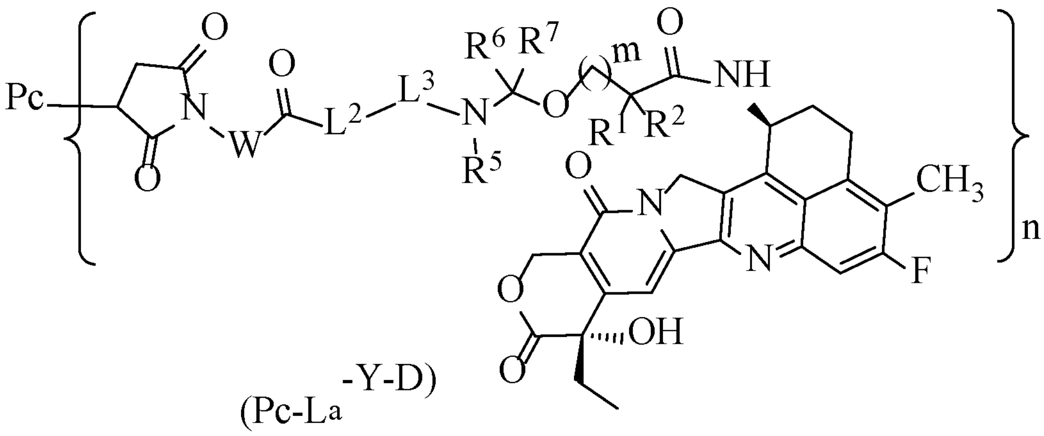

(Pc-$L_a$-Y-D)

wherein,

Pc is the isolated anti-HER3 antibody according to any one of claims 1 to 8;
m is an integer from 0 to 4;
n is a decimal or an integer from 1 to 10;
$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with the carbon atom to which they are attached, form cycloalkyl or heterocyclyl;
W is selected from the group consisting of $C_{1-8}$ alkyl, $C_{1-8}$ alkyl-$C_{3-6}$ cycloalkyl and linear heteroalkyl of 1 to 8 chain atoms, and the linear heteroalkyl of 1 to 8 chain atoms comprises 1 to 3 heteroatoms selected from the group consisting of N, O and S, wherein the $C_{1-8}$ alkyl, $C_{1-8}$ alkyl-$C_{3-6}$ cycloalkyl and linear heteroalkyl of 1 to 8 chain atoms are each independently optionally further substituted with one or more substituents selected from the group consisting of halogen, hydroxy, cyano, amino, alkyl, chloroalkyl, deuterated alkyl, alkoxy and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;
$R^5$ is selected from the group consisting of hydrogen, alkyl, haloalkyl, deuterated alkyl and hydroxyalkyl;
$R^6$ and $R^7$ are identical or different and are each independently selected from the group consisting of hydrogen, halogen, alkyl, haloalkyl, deuterated alkyl and hydroxyalkyl.

**14.** The antibody-drug conjugate of general formula (Pc-$L_a$-Y-D) or the pharmaceutically acceptable salt thereof according to claim 13, wherein the antibody-drug conjugate is:

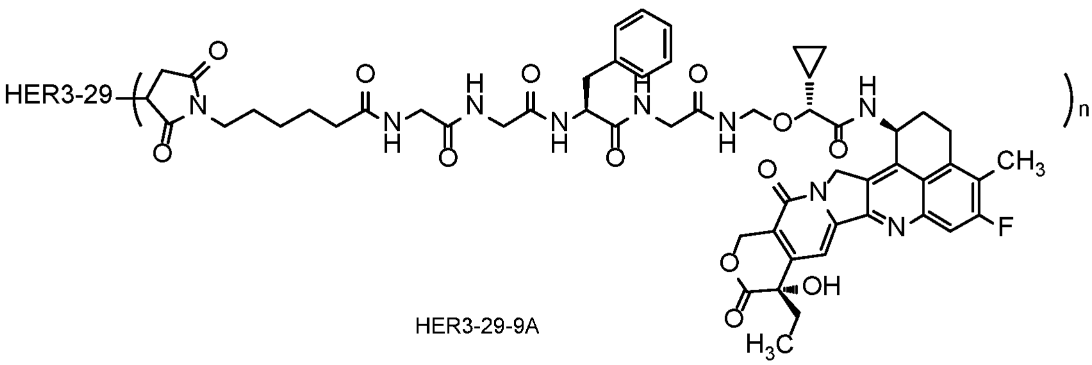

HER3-29-9A

wherein:

n is a decimal or an integer from 1 to 8; preferably, n is a decimal or an integer from 3 to 8;
HER3-29 is an anti-HER3 antibody comprising a heavy chain set forth in SEQ ID NO: 27 and a light chain set forth in SEQ ID NO: 28.

**15.** A method for preparing the antibody-drug conjugate of general formula ($Pc-L_a-Y-D$) or the pharmaceutically acceptable salt thereof according to claim 13, comprising the following step:

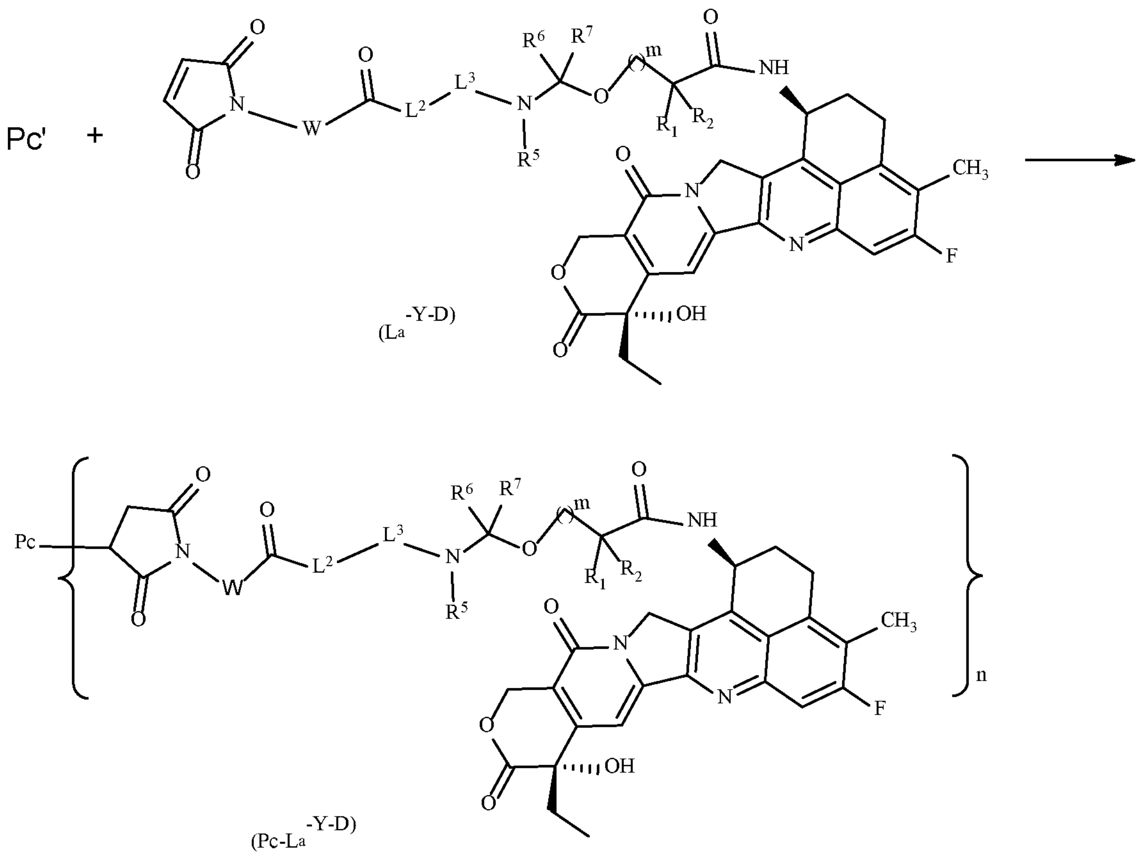


conducting a coupling reaction of Pc' with a compound of general formula ($L_a$-Y-D) to give a compound of general formula (Pc-$L_a$-Y-D);
wherein:
Pc' is obtained by reducing Pc; Pc, n, m, W, $L^2$, $L^3$, $R^1$, $R^2$, $R^5$, $R^6$ and $R^7$ are as defined in claim 13.

**16.** A pharmaceutical composition comprising the isolated anti-HER3 antibody according to any one of claims 1 to 8, or the nucleic acid molecule according to claim 9, or the antibody-drug conjugate or the pharmaceutically acceptable salt thereof according to claim 13 or 14, and one or more pharmaceutically acceptable excipients, diluents or carriers.

**17.** Use of the isolated anti-HER3 antibody according to any one of claims 1 to 8, or the nucleic acid molecule according to claim 9, or the antibody-drug conjugate or the pharmaceutically acceptable salt thereof according to claim 13 or 14, or the pharmaceutical composition according to claim 16, in preparing a medicament for treating a HER3-mediated disease or disorder.

**18.** Use of the isolated anti-HER3 antibody according to any one of claims 1 to 8, or the nucleic acid molecule according to claim 9, or the antibody-drug conjugate or the pharmaceutically acceptable salt thereof according to claim 13 or 14, or the pharmaceutical composition according to claim 16, in preparing a medicament for treating and/or preventing a tumor, wherein
preferably, the tumor is selected from the group consisting of breast cancer, non-small cell lung cancer, gastric cancer, ovarian cancer, prostate cancer, bladder cancer, colorectal cancer, head and neck squamous cell carcinoma, and melanoma.

**19.** A kit comprising the isolated anti-HER3 antibody according to any one of claims 1 to 8, or the nucleic acid molecule according to claim 9, or the antibody-drug conjugate or the pharmaceutically acceptable salt thereof according to claim 13 or 14, or the pharmaceutical composition according to claim 16.

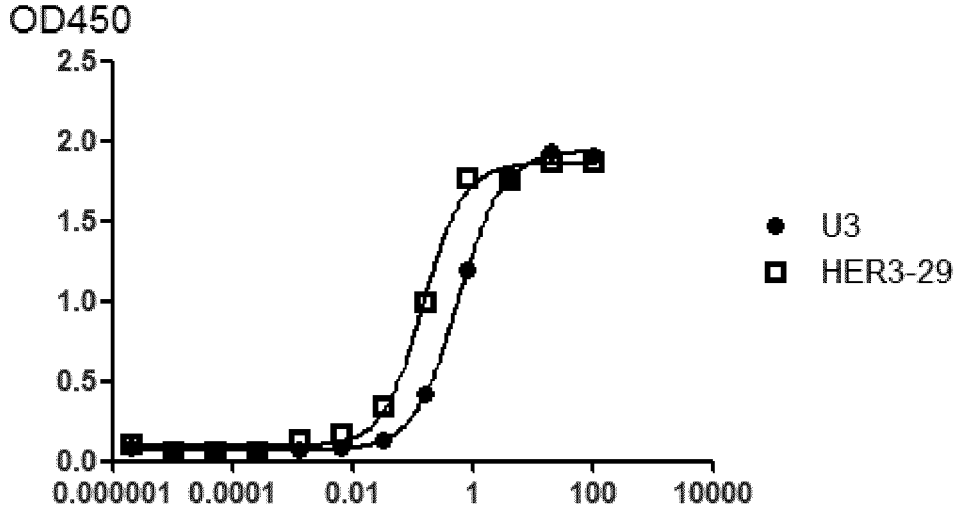
OD450
2.5
2.0
1.5
1.0
0.5
0.0
0.000001
0.0001
0.01
1
100
10000
U3
HER3-29

FIG. 1

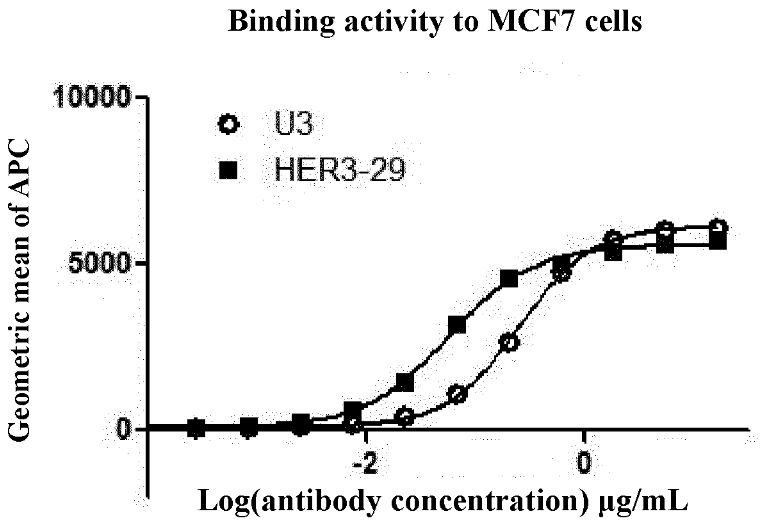
Binding activity to MCF7 cells
Geometric mean of APC
10000
5000
0
-2
0
U3
HER3-29
Log(antibody concentration) µg/mL

FIG. 2

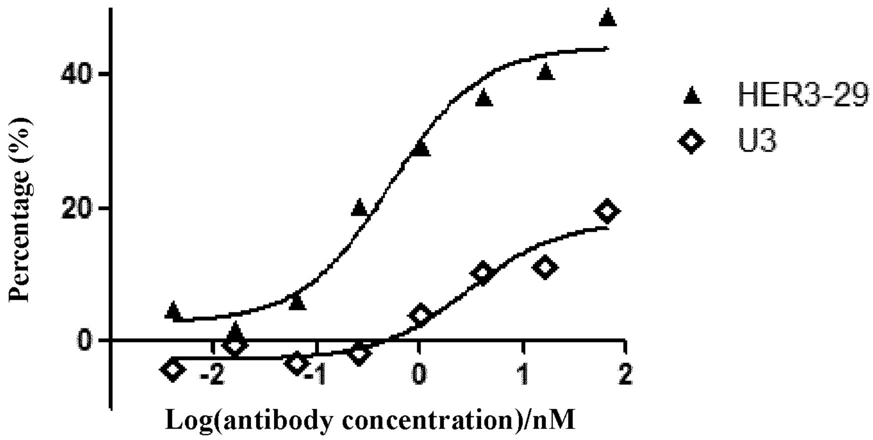
40
20
0
Percentage (%)
-2
-1
0
1
2
Log(antibody concentration)/nM
HER3-29
U3

FIG. 3

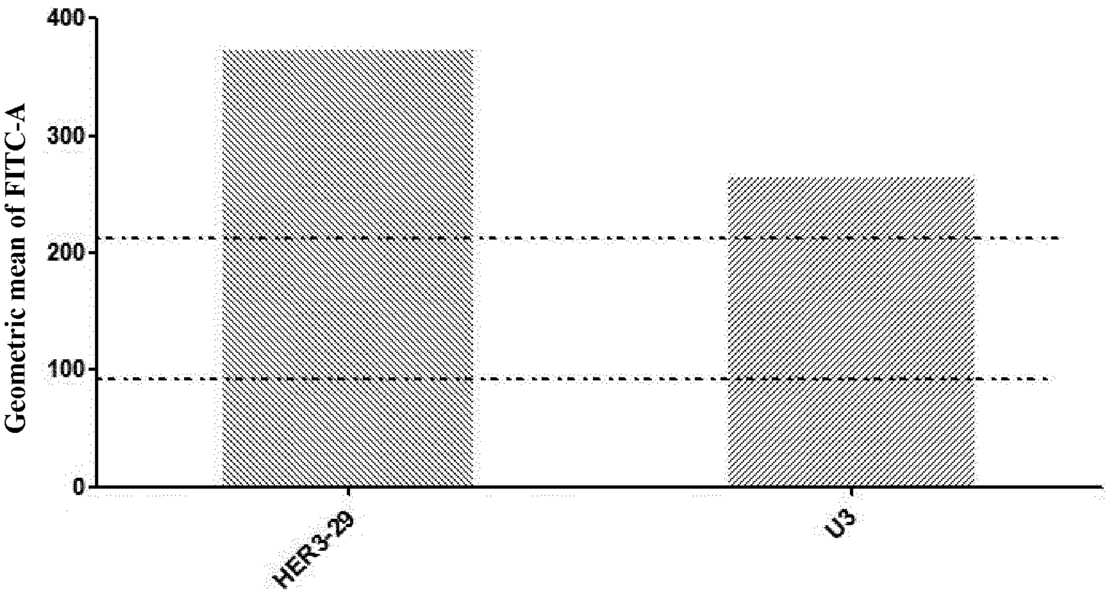
400
300
200
100
0
Geometric mean of FITC-A
HER3-29
U3

FIG. 4

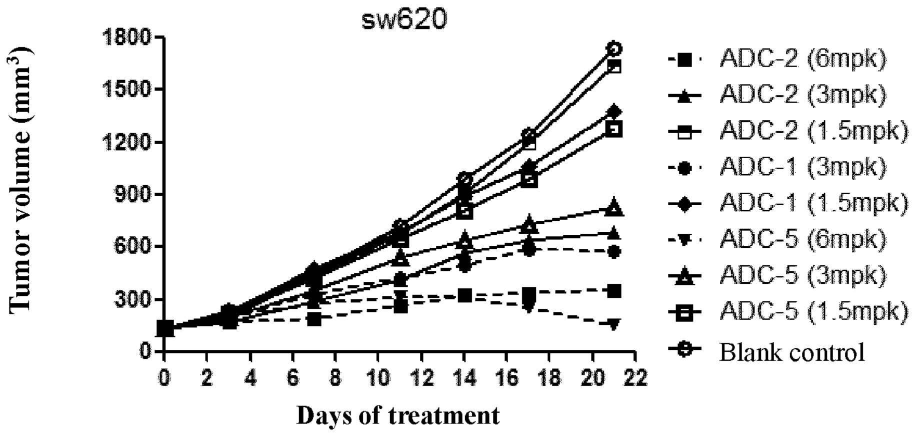
sw620
Tumor volume (mm3)
1800
1500
1200
900
600
300
0
0 2 4 6 8 10 12 14 16 18 20 22
Days of treatment
ADC-2 (6mpk)
ADC-2 (3mpk)
ADC-2 (1.5mpk)
ADC-1 (3mpk)
ADC-1 (1.5mpk)
ADC-5 (6mpk)
ADC-5 (3mpk)
ADC-5 (1.5mpk)
Blank control

FIG. 5

**INTERNATIONAL SEARCH REPORT**

International application No. **PCT/CN2021/123733**

**A. CLASSIFICATION OF SUBJECT MATTER**

C07K 16/28(2006.01)i; A61K 47/65(2017.01)i; A61K 47/68(2017.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

SIPOABS; CNABS; DWPI; CNKI; ISI web of Science; Wanfang; Genbank: 表皮生长因子受体3, 抗体偶联药物, 依喜替康, 内吞, 结合, applicant/inventor, ERBB3, HER3, EGFR3, antibody, drug conjugate, binding, internalize, exatecan, DX derivate, SEQ ID NO: 7, 8

**C. DOCUMENTS CONSIDERED TO BE RELEVANT**

| Category* | Citation of document, with indication, where appropriate, of the relevant passages | Relevant to claim No. |
|---|---|---|
| X | CN 105120890 A (SORRENTO THERAPEUTICS INC.) 02 December 2015 (2015-12-02) claims 1-14, description paragraphs 58, 62, 64-68, 103-109, 160-163, embodiments 1-2, 8, sequence table | 1, 4-12, 16-19 |
| Y | CN 105120890 A (SORRENTO THERAPEUTICS INC.) 02 December 2015 (2015-12-02) claims 1-14, description paragraphs 58, 62, 64-68, 103-109, 160-163, embodiments 1-2, 8, sequence table | 13, 15-19 |
| Y | CN 112912109 A (DAIICHI SANKYO COMPANY, LIMITED) 04 June 2021 (2021-06-04) claims 8-34 | 13, 15-19 |
| Y | WO 2020063676 A1 (JIANGSU HENGRUI MEDICINE CO., LTD. et al.) 02 April 2020 (2020-04-02) claims 20-26 | 13, 15-19 |
| Y | CN 110475569 A (KINKI UNIVERSITY et al.) 19 November 2019 (2019-11-19) claims 8-11, 26-27 | 13, 15-19 |
| A | CN 105367657 A (SHANGHAI INSTITUTE OF BIOLOGICAL PRODUCTS CO., LTD.) 02 March 2016 (2016-03-02) entire document | 1-19 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 December 2021** | **17 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/123733**

**C. DOCUMENTS CONSIDERED TO BE RELEVANT**

| Category* | Citation of document, with indication, where appropriate, of the relevant passages | Relevant to claim No. |
|---|---|---|
| A | CN 103002912 A (MERRIMACK PHARMACEUTICALS INC.) 27 March 2013 (2013-03-27)<br>entire document | 1-19 |
| A | CN 105209493 A (BOARD OF REGENTS, THE UNIVERSITY OF TEXAS SYSTEM) 30 December 2015 (2015-12-30)<br>entire document | 1-19 |
| A | US 2017101480 A1 (MERRIMACK PHARMACEUTICALS, INC.) 13 April 2017 (2017-04-13)<br>entire document | 1-19 |
| A | US 2015210766 A1 (SAMSUNG ELECTRONICS CO., LTD.) 30 July 2015 (2015-07-30)<br>entire document | 1-19 |
| A | WO 2020015687 A1 (SHANGHAI INSTITUTE OF BIOLOGICAL PRODUCTS CO., LTD.) 23 January 2020 (2020-01-23)<br>entire document | 1-19 |
| A | CN 106794258 A (JIANGSU HENGRUI MEDICINE CO., LTD. et al.) 31 May 2017 (2017-05-31)<br>entire document | 1-19 |
| Y | 刘连奇 等 (LIU, Lianqi et al.). "靶向HER3的在研抗体偶联药物-U3-1402 (HER3-targeting antibody-drug conjugate——U3-1402)"<br>*临床药物治疗杂志 (Clinical Medication Journal),*<br>Vol. 17, No. 9, 30 September 2019 (2019-09-30),<br>pp. 1-4 | 13, 15-19 |
| A | GANDULLO-SANCHEZ, L. et al. "HER3 targeting with an antibody-drug conjugate bypasses resistance to anti-HER2 therapies"<br>*EMBO MOLECULAR MEDICINE,* Vol. 12, 24 April 2020 (2020-04-24),<br>document, no. e11498 | 1-19 |

Form PCT/ISA/210 (second sheet) (January 2015)

**INTERNATIONAL SEARCH REPORT**

International application No.
**PCT/CN2021/123733**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/CN2021/123733**

| Patent document cited in search report | Publication date (day/month/year) | Patent family member(s) | Publication date (day/month/year) |
|---|---|---|---|
| CN 105120890 A | 02 December 2015 | JP 2015534997 A | 07 December 2015 |
| | | WO 2014066530 A2 | 01 May 2014 |
| | | CN 105120890 B | 19 January 2018 |
| | | EP 2912066 A2 | 02 September 2015 |
| | | HK 1214283 A1 | 22 July 2016 |
| | | EP 2912066 A4 | 12 October 2016 |
| | | US 2016311923 A1 | 27 October 2016 |
| | | US 9346890 B2 | 24 May 2016 |
| | | US 9938352 B2 | 10 April 2018 |
| | | CA 2928539 A1 | 01 May 2014 |
| | | US 2014120092 A1 | 01 May 2014 |
| | | WO 2014066530 A3 | 16 July 2015 |
| CN 112912109 A | 04 June 2021 | KR 20210062005 A | 28 May 2021 |
| | | CA 3113207 A1 | 26 March 2020 |
| | | WO 2020059772 A1 | 26 March 2020 |
| | | TW 202024133 A | 01 July 2020 |
| WO 2020063676 A1 | 02 April 2020 | AU 2019349207 A1 | 08 April 2021 |
| | | TW 202027795 A | 01 August 2020 |
| | | CA 3114137 A1 | 02 April 2020 |
| | | BR 112021004656 A2 | 01 June 2021 |
| | | KR 20210066833 A | 07 June 2021 |
| | | CN 112512591 A | 16 March 2021 |
| CN 110475569 A | 19 November 2019 | KR 20190120764 A | 24 October 2019 |
| | | CA 3053749 A1 | 07 September 2018 |
| | | EP 3590534 A4 | 23 December 2020 |
| | | TW 201834696 A | 01 October 2018 |
| | | US 2020061031 A1 | 27 February 2020 |
| | | SG 11201907050 P A | 27 September 2019 |
| | | JP WO2018159582 A1 | 19 December 2019 |
| | | EP 3590534 A1 | 08 January 2020 |
| | | BR 112019015915 A2 | 07 April 2020 |
| | | AU 2018227146 A1 | 29 August 2019 |
| | | WO 2018159582 A1 | 07 September 2018 |
| CN 105367657 A | 02 March 2016 | CN 105367657 B | 13 September 2019 |
| | | CN 110642952 B | 25 May 2021 |
| | | CN 110642952 A | 03 January 2020 |
| CN 103002912 A | 27 March 2013 | JP 5752687 B2 | 22 July 2015 |
| | | JP 2013506622 A | 28 February 2013 |
| | | MX 2012002172 A | 29 May 2012 |
| | | MA 33582 B1 | 01 September 2012 |
| | | AU 2010284018 B2 | 05 June 2014 |
| | | CA 2771744 A1 | 24 February 2011 |
| | | PE 20121585 A1 | 29 November 2012 |
| | | WO 2011022727 A2 | 24 February 2011 |
| | | IN 1518DEN2012 A | 05 June 2015 |
| | | WO 2011022727 A3 | 27 June 2013 |
| | | ZA 201201195 B | 29 July 2015 |
| | | TN 2012000057 A1 | 19 September 2013 |
| | | EC SP12011740 A | 28 February 2013 |

Form PCT/ISA/210 (patent family annex) (January 2015)

**INTERNATIONAL SEARCH REPORT**
**Information on patent family members**

International application No.
**PCT/CN2021/123733**

| Patent document cited in search report | | | Publication date (day/month/year) | Patent family member(s) | | | Publication date (day/month/year) |
|---|---|---|---|---|---|---|---|
| | | | | BR | 112012003809 | A2 | 24 September 2019 |
| | | | | DO | P2012000044 | A | 30 June 2012 |
| | | | | AU | 2010284018 | C1 | 15 October 2015 |
| | | | | KR | 20120059568 | A | 08 June 2012 |
| | | | | IL | 218097 | D0 | 30 April 2012 |
| | | | | SG | 178509 | A1 | 27 April 2012 |
| | | | | MX | 336091 | B | 08 January 2016 |
| | | | | AU | 2010284018 | A1 | 22 March 2012 |
| | | | | NI | 201200027 | A | 29 January 2013 |
| | | | | EA | 201200195 | A1 | 28 December 2012 |
| | | | | EP | 2467164 | A2 | 27 June 2012 |
| | | | | CR | 20120108 | A | 05 June 2012 |
| CN | 105209493 | A | 30 December 2015 | US | 9725520 | B2 | 08 August 2017 |
| | | | | CN | 105209493 | B | 03 May 2019 |
| | | | | EP | 2970494 | A1 | 20 January 2016 |
| | | | | US | 10358501 | B2 | 23 July 2019 |
| | | | | US | 2018066066 | A1 | 08 March 2018 |
| | | | | EP | 2970494 | A4 | 14 September 2016 |
| | | | | WO | 2014159915 | A1 | 02 October 2014 |
| | | | | US | 2016032011 | A1 | 04 February 2016 |
| | | | | EP | 2970494 | B1 | 13 December 2017 |
| | | | | US | 2020055954 | A1 | 20 February 2020 |
| US | 2017101480 | A1 | 13 April 2017 | US | 10184006 | B2 | 22 January 2019 |
| US | 2015210766 | A1 | 30 July 2015 | US | 9505843 | B2 | 29 November 2016 |
| | | | | KR | 102127408 | B1 | 29 June 2020 |
| | | | | KR | 20150090761 | A | 06 August 2015 |
| WO | 2020015687 | A1 | 23 January 2020 | EP | 3825334 | A1 | 26 May 2021 |
| | | | | CN | 110724194 | B | 19 March 2021 |
| | | | | CN | 110724194 | A | 24 January 2020 |
| CN | 106794258 | A | 31 May 2017 | BR | 112017016503 | A2 | 10 April 2018 |
| | | | | CN | 111150851 | A | 15 May 2020 |
| | | | | KR | 20170117473 | A | 23 October 2017 |
| | | | | EP | 3251698 | A4 | 17 October 2018 |
| | | | | MX | 2017010102 | A | 23 November 2017 |
| | | | | RU | 2708461 | C2 | 09 December 2019 |
| | | | | US | 2018177890 | A1 | 28 June 2018 |
| | | | | CN | 106794258 | B | 12 July 2019 |
| | | | | EP | 3251698 | A1 | 06 December 2017 |
| | | | | TW | I704929 | B | 21 September 2020 |
| | | | | AU | 2016218840 | A1 | 31 August 2017 |
| | | | | RU | 2017131158 | A3 | 17 July 2019 |
| | | | | WO | 2016127790 | A1 | 18 August 2016 |
| | | | | TW | 201628658 | A | 16 August 2016 |
| | | | | RU | 2017131158 | A | 15 March 2019 |
| | | | | CA | 2976050 | A1 | 18 August 2016 |
| | | | | JP | 2018512377 | A | 17 May 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5500362 A
- US 5821337 A
- US 4683195 A
- CN 101573384
- WO 2016127790 A1
- US 20050238649 A1
- US 5208020 A
- WO 2007077028 A2
- WO 2020063676 A1
- WO 2020063676 A
- WO 2015155998 A1


### Non-patent literature cited in the description


- *J. biol. chem,* 1968, vol. 243, 3558
- **BIRD et al.** *Science,* 1988, vol. 242, 423-426
- **HUSTON et al.** *Proc. Natl. Acad. Sci USA,* 1988, vol. 85, 5879-5883
- **KABAT et al.** Sequences of Proteins of Immunological Interest. Public Health Service, National Institutes of Health, 1991
- **MARTIN.** *ACR. Protein Sequence and Structure Analysis of Antibody Variable Domains,* 2001
- **LEFRANC, M.P.** *Dev. Comp. Immunol.,* 2003, vol. 27, 55-77
- Epitope Mapping Protocols in Methods in Molecular Biology. 1996, vol. 66
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies: A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82
- **CLYNES et al.** *PNAS USA,* 1998, vol. 95, 652-656
- **VAN BIJ et al.** *Journal of Hepatology,* October 2010, vol. 53 (4), 677-685
- Antibodies: A Laboratory Manual. Cold Spring Harbor Press
- *immunoglobulin journal,* 2001, ISBN ISBN012441351
- **WATSON et al.** Molecular Biology of the Gene. The Benjamin/Cummings Pub. Co, 1987, 224
- **ALTSCHUL, S.F. et al.** *J. Mol. Biol.,* 1990, vol. 215, 403-410
- **GISH, W et al.** *Nature Genet.,* 1993, vol. 3, 266-272
- **MADDEN, T.L. et al.** *Meth. Enzymol.,* 1996, vol. 266, 131-141
- **ALTSCHUL, S.F. et al.** *Nucleic Acids Res.,* 1997, vol. 25, 3389-3402
- **ZHANG, J. et al.** *Genome Res.,* 1997, vol. 7, 649-656
- **MULLIS et al.** Quant. Biol. Cold Spring Harbor Symp, 1987, vol. 51, 263
- PCR TECHNOLOGY. Stockton Press, N.Y, 1989
- **T.W.GREENE ; P.G.M.WUTS.** Protective Groups in Organic Synthesis
- **CHARI et al.** *Cancer Research,* 1992, vol. 52, 127-131
- **SAMBROOK et al.** Molecular Cloning: A Laboratory Manual. Cold Spring Harbor Laboratory Press
- **AUSUBEL et al.** Current Protocols in Molecular Biology. Greene Publishing Association, Wiley Interscience